# EUROPEAN PATENT APPLICATION

(11) **EP 4 332 089 A1**
(43) Date of publication of application: **06.03.2024**
(21) Application number: 22193497.9
(22) Date of filing: 01.09.2022
(51) Int. Cl.: C07C 271/22, C07C 269/06, C07D 201/00, C07D 313/12, C07D 317/60, C07D 333/24, C07J 1/00

(54) **METHOD FOR PREPARING AMINO ACIDS**

(71) Applicant: PHILIPPS-UNIVERSITÄT MARBURG, 35037 Marburg (DE)
(72) Inventor: Meggers, Eric, 35039 Marburg (DE); Ye, Chen-Xi, 35039 Marburg (DE)
(74) Representative: Durm Patentanwälte PartG mbB

(57) **Abstract**

Described is a method for preparing α-amino acids and α-deuterated α-amino acids, comprising the step of reacting a carboxylic acid with a carbamate of the formula PG-NH-X, wherein PG is a protecting group and X is a leaving group, in the presence of a catalyst, wherein the catalyst provides an enantioselective single-step α-amination of the carboxylic acid. Furthermore, the use of said catalyst in this method is described.

## Description

The present invention relates to a method for preparing amino acids and the use of a catalyst in this method.

α-Amino acids are essential biomolecules that serve as building blocks for proteins, peptides and bioactive metabolites, among others. In addition, α-amino acids are broadly used as chiral feedstocks for the synthesis of chiral molecules. Beyond the set of proteinogenic and natural non-proteinogenic amino acids used by Nature, α-amino acids bearing unnatural side chains with modulated chemical, physical and pharmaceutical properties are sought-after synthetic building blocks (1-4). For example, Paxlovid, a drug developed recently by Pfizer for COVID-19 treatment, contains a peptidic protease inhibitor including two unusual α-amino acid units (5). Despite the increasing demand for α-amino acids and the many methods that have been developed for their preparation, the efficient and catalytic asymmetric synthesis of unnatural α-amino acids remains a challenge (*6, 7*). Current methods often require multi-step synthetic sequences, employ inconvenient starting materials, or involve protecting groups that are difficult to cleave.

A desirable approach for the synthesis of α-amino acids is the direct α-amination of carboxylic acids, which are abundant feedstock molecules. However, while the α-amination of aldehydes, ketones, 1,3-dicarbonyl compounds, esters, and some carboxylic acid surrogates is well-established, direct α-amination of carboxylic acids remains sparse and highly challenging (*8-10*). This can be attributed to both the acidity of the carboxylic acid moiety and the typically high p*K*ₐ value of the α-hydrogen. In 1972, Yamada was the first to report the preparation of α-amino acids from carboxylic acids by a one-pot procedure through double deprotonation followed by reaction with O-methylhydroxylamine (*11*). However, this method requires a strong base and provides only racemic products. In 2012, Smith reported the enantioselective organocatalytic α-amination of carboxylic acids with *N*-aryl-*N*-aroyldiazenes in a one-pot, two-step procedure via in situ generated acid anhydrides (12). This method is limited to the synthesis of *N*-aryl-α-glycine derivatives after SmI₂-induced cleavage of the initially obtained hydrazides. In 2019, Shimizu introduced a boron-catalyzed α-hydrazination of carboxylic acids with diisopropyl azodicarboxylate (*13*). In one example, the presence of a chiral ligand provided modest enantioselectivity (45% ee). Unfortunately, converting the α-hydrazino into an amino group required harsh reaction conditions.

Transition metal catalysis has proven a powerful tool for the amination of non-activated C(sp³)-H bonds (*14, 15*)*.* Directed C-H activation using a metal-coordinating directing group enables the site-selective activation of a specific C-H bond through the formation of a cyclometalated intermediate, followed by the reaction of the metal-carbon bond with an external or metal-bound nitrogen species. Due to the requirement for non-strained metallacycle formation, the coordinating atom is usually at least 3-bonds away from the activated C-H. A further drawback is the reliance on noble metals such as Pd, Ru, Rh, and Ir for inducing cyclometalation. In a different mechanistic manifold, transition metals can catalyze the insertion of nitrenes into C-H bonds in a stepwise or concerted fashion. While such nitrene-mediated C(sp³)-H aminations are very powerful due to the high reactivity of the intermediate transition metal nitrenoids, control of regio- and stereochemistry poses challenges for intermolecular reactions in which the substrate is not bound to the metal as the final C-N bond formation step must occur through an outer-sphere mechanism (*15, 16*).

Inspired by the progress in directed C-H activation and nitrene-mediated C-H functionalizations, it was sought to merge these two mechanistic modes and to identify a mechanism to achieve an asymmetric intermolecular vicinal C(sp³)-H amination through a directed C(sp³)-H nitrene insertion. Directed nitrene insertions into C-H bonds without the preceding formation of a C-M bond have only been realized in racemic reactions through high-valent iridium (*17*) and rhodium (18) nitrene intermediates, and not in a catalytic asymmetric manner. Specifically, it was envisioned that carboxylic acids might be suitable directing groups for regioselective and stereocontrolled nitrene-mediated C-H aminations to directly convert abundant feedstock carboxylic acids into highly valuable enantioenriched α-amino acids.

Currently used synthesis methods are time-consuming, not efficient, generate more waste products and are therefore also expensive overall. The factors of time consumption, waste generation and synthesis cost are of great importance considering the wide application of α-amino acids in the pharmaceutical industry and biotechnology.

Thus, the technical problem underlying the present invention is to enable the synthesis of α-amino acids, wherein the drawbacks of the prior art are overcome.

This technical problem has been solved by the subject-matter of the independent claims. Preferred embodiments are defined in the dependent claims.

According to the present invention, there is provided a method for preparing α-amino acids and α-deuterated α-amino acids, comprising the step of reacting a carboxylic acid with a carbamate of the formula PG-NH-X, wherein PG is a protecting group and X is a leaving group, in the presence of a catalyst, wherein the catalyst provides an enantioselective singe-step α-amination of the carboxylic acid.

The method according to the present invention can be illustrated by the following reaction scheme:

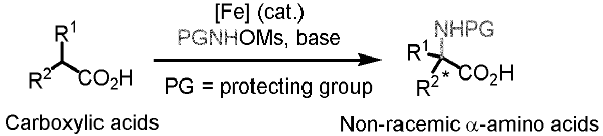

The protecting group PG remains in the obtained α-amino acid. It can be later removed depending on the particular further use of the α-amino acid. That is, if for the subsequent reactions and use of the α-amino acid the protection of the N-atom is required, the protecting group can be remained. If the protective group is cleaved, this can be carried out in a manner known in the prior art.

In one embodiment of the method according to the present invention, the leaving group can be a sulfonate, for example it can be a sulfonate selected from the group consisting of toluenesulfonate, isopropylsulfonate and mesylate.

In a further embodiment, the protecting group is an electron withdrawing protecting group, preferably a carbamate moiety. For example, the electron withdrawing protecting group is selected from the group consisting of the CO₂Me group, the CO₂CH₂CCl₃ (Troc) group, the tert-butyloxycarbonyl (Boc) group, the benzyloxycarbonyl (Cbz) group, the allyloxycarbonyl (Alloc) group, and other amine protecting group that form a carbamate.

The method according to the present invention is applicable to a wide range of carboxylic acids having at least one hydrogen at the α-C-atom and one or two groups, also called substituents, bound to the α-C-atom. Such carboxylic acids can be formally represented by the general formula R-CH₂-C(O)OH (a single group R bound to the α-C-atom) or R¹R²CH-C(O)OH (two groups R¹ and R² bound to the α-C-atom, also called a α-branched carboxylic acid). In one embodiment, the carboxylic acid has a group R at the α-C-atom containing a residue selected from the group consisting of aryl, allyl, vinyl, propargyl, alkyl moieties, or other side chains known in this technical field. In another embodiment, the carboxylic acid is branched in α-position and has two side chains R¹ and R² selected independently from the group consisting of aryl, allyl, vinyl, propargyl, alkyl moieties, or other known side chains known in this field. This means that the groups R, R¹ and R² in the above formula have a group derivable from aryl, allyl, vinyl, propargyl, alkyl moieties, or other side chains known in this field, wherein further groups, atoms and substituent can be present depending on the intended further use of the obtained α-amino acids.

As pointed out above, the method according to the present invention is carried out in the presence of a catalyst. Every catalyst can be employed in the method according to the present invention, which can provide the enantioselective singe-step α-amination of the carboxylic acid to generate α-amino acids. Furthermore, every chiral non-racemic catalyst can be employed in the method according to the present invention, which can provide the above-mentioned enantioselective singe-step α-amination of the carboxylic acid in a stereocontrolled fashion to generate non-racemic α-amino acids. For example, the catalyst is a chiral non-racemic iron catalyst. In one embodiment, the chiral non-racemic iron catalyst is a chiral non-racemic iron (II) catalyst containing organic ligands. In the catalyst, for example in the chiral iron (II) catalyst, the central metal can have six coordination sites to which ligands can bind to. In one embodiment the catalyst contains two monodentate ligands, in particular wherein the monodentate ligands are selected from the group consisting of acetonitrile, triflate or other sulfonates, acetate or other carboxylates, chloride, bromide, iodide, pseudohalides, and other potentially labile monodentate ligands. The two monodentate ligands can be identical or different from each other. These two monodentate ligands, which can be removable from the catalyst, provide two binding sites at the catalyst.

Furthermore, the catalyst can contain one tetradentate ligand, which can contain four N-atoms to bind to the central atom, in particular Fe. Further, the catalyst can contain cyclic organic residue in which these N-atoms are contained as heteroatoms.

Other chiral non-racemic transition metal catalysts which are known in the prior art are also suitable for catalyzing the enantioselective singe-step α-amination of the carboxylic acid, such as catalysts with metals selected from manganese, cobalt, nickel, copper, rhodium and iridium in various oxidation states with monodentate, bidentate, tridentate, and/or tetradentate ligands or a combination thereof.

Other transition metal catalysts which are known in the prior art are suitable for catalyzing the enantioselective singe-step α-amination of the carboxylic acid in a non-stereocontrolled fashion, thereby providing racemic α-amino acids.

In one embodiment the catalyst is selected from the group consisting of *(R,R)*-Fe1, *(R,R)*-Fe2 and *(R,R)*-FeBIP.
*(R,R)*-Fe1: bis(acetonitrile)[(2*R*,2'*R*)-1, 1'-bis(pyridin-2-ylmethyl)-2,2'-bipyrrolidine-κ⁴*N*]iron(II) bis(hexafluoroantimonate)
(*R*,*R*)-Fe2: bis(acetonitrile)[(2*R*,2'*R*)-1,1'-bis({5-[2,6-bis(trifluoromethyl)phenyl]pyridin-2-yl}methyl)-2,2'-bipyrrolidine-κ⁴*N*]iron(II) bis(hexafluoroantimonate)
(*R*,*R*)-FeBIP: dichlorido{(2*R*,2'*R*)-1,1'-bis[(1-methyl-1*H*-benzo[*d*]imidazol-2-yl)methyl]-2,2'-bipyrrolidine-κ⁴*N*}iron(II)] or its enantiomer (*S*,*S*)-FeBIP,

The reaction conditions for carrying out the method according to the present invention are chosen so that the desired α-amino acid is sufficiently provided. For example, the amount of catalyst can be 0.2 mol% to 15 mol%, referred to the amount of the carboxylic acid, for example 1 mol% to 8 mol%, in particular about 8 mol%. The reaction temperature is chosen so that the reaction is sufficiently completed, the amount of side products is not too high and the enatioselectivity is sufficient. The reaction temperature can for example be -25 °C to 25 °C, for example -15 °C to 0 °C, in particular about -15 °C. The method according to the present invention can be carried out in a solvent. The solvent is selected so that the educts and the other reaction constituents are dissolved therein. The solvent can be an aprotic solvent. Examples of suitable solvents are halogenated hydrocarbons such as methylene chloride, dichloromethane, 1,2-dichloroethane, chloroform, chlorobenzene, 1,2-dichlorobenzene, and 1,2-difluorobenzene. Other solvents, which can be employed in the method according to the present invention are tetrahydrofuran, toluene, ethyl acetate, acetone, acetonitrile, dimethylformamide, among others. Omitting the solvent is also feasible. Furthermore, the method according to the present invention can be carried out in the presence of a base comprising for example K₂CO₃, KHCO₃, piperidine, 2,2,6,6-tetramethylpiperidine, or Et₃N. The amount of the base employed in the method according to the present invention can be 2 equivalents to 5 equivalents, in particular about 4 equivalents, of the base to 1 equivalent of the carboxylic acid. Other amounts of base are also feasible. The amount of the carbamate of the formula PG-NH-X can be 1 equivalent to 4 equivalent, in particular about 2 equivalents, referred to 1 equivalent of the carboxylic acid. The reaction time is chosen so that the reaction is sufficiently completed. The reaction time can be 1 h to 24 h, for example 8 h to 24 h, in particular about 20 h.

As pointed out above, the α-amino acid obtained by the method according to the present invention contains the protecting group. If required depending on the further use of the α-amino acid the protecting group can be cleaved off.

Furthermore, the α-amino acid, which may contain the protecting group, can be recrystallized so that the enantiopurity can be increased. An example of a solvent to be used in the recrystallization can be a mixture of ethanol and n-hexane.

Subject-matter of the present invention is further the use of a catalyst, in particular the catalyst as defined above, for carrying out the enantioselective singe-step α-amination of the carboxylic acid. For the details of the use of the catalyst and the catalyst itself it is referred to the above detailed description of the method according to the present invention in its entirety, in particular the method steps and the employed materials.

To be more specific for illustrative purpose, the present invention involves a particularly efficient method for the synthesis of chiral nonracemic α-amino acids. The method according to the present invention is a one-step synthesis of nonracemic α-amino acids starting from carboxylic acids by a stereocontrolled direct amination. This method is generally applicable to carboxylic acids and very efficient. The key step is the direct stereocontrolled amination of carboxylic acids in the α-position. Thus, starting from simple carboxylic acids, the corresponding nitrogen-protected amino acid can be prepared in a single synthetic step.

Initially selected was the carboxylic acid phenylacetic acid (PAA) as a model substrate. (*R*,*R*)-[FeCl₂(BIP)], bearing a bis-benzimidazole ligand with a chiral 2,2'-bipyrrolidine backbone (BIP), hereafter referred to as (*R*,*R*)-FeBIP, was chosen as the catalyst as it was recently identified by us as an excellent catalyst for a 1,3-migratory nitrene insertion (Table 1) (19). The coordination of the deprotonated PAA and the simultaneous formation of an iron nitrenoid triggers a nitrene-mediated amination of the α-C(sp³)-H bond to form phenylglycine via a cyclic transition state with potentially high regio- and stereocontrol. As protecting group, the *tert*-butyloxycarbonyl (Boc) group was elected, one of the most commonly employed *N*-protecting groups in amino acid chemistry (20).

The suitability of *N*-Boc-protected carbamates was explored with various leaving groups at the nitrogen (BocNH-X, X = leaving group) as nitrene precursors (21-26). With toluenesulfonate as the leaving group, the desired *N*-Boc-protected phenylglycine was formed with 29% NMR yield and 91% ee after 20 hours at-15 °C in an incomplete conversion (entry 1). An isopropylsulfonate leaving group provided similar results (entry 2). The best results were obtained with mesylate as the leaving group. Subjecting PAA to (*R*,*R*)-FeBIP (8 mol%), BocNHOMs (2 equiv) and piperidine (4 equiv) in 1,2-dichlorobenzene (o-DCB) at -15 °C provided *N*-Boc-protected (*S*)-phenylglycine (compound 1) in 78% NMR yield (77% isolated yield) with 93% ee (entry 3). Other bases such as K₂CO₃ (entry 4) or Et₃N (entry 5) were also employable. Other solvents, including dichloromethane or acetonitrile, can also be used (entries 6 and 7). It is worth noting that higher temperatures also led to diminished yields (entries 8 and 9), presumably due to the Lossen-type rearrangement of BocNHOMs induced by base at elevated temperatures (27).

**Table 1.**

| 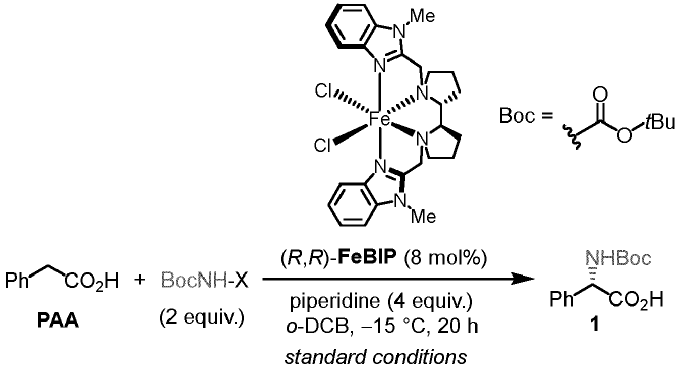 | | | | | | |
|---|---|---|---|---|---|---|
| Entry | X^{∗} | Reaction conditions^{†} | Temp. (°C) | Conv. (%)^{‡} | Yield (%)^{‡} | ee (%)^{‡} |
| 1 | OTs | standard conditions | -15 | 35 | 29 | 91 |
| 2 | OSO₂*i*Pr | standard conditions | -15 | 44 | 31 | 93 |
| 3 | OMs | standard conditions | -15 | 90 | 78 (77)^{§} | 93 |
| 4 | OMs | K₂CO₃ as base | -15 | 31 | 7 | 93 |
| 5 | OMs | Et₃N as base | -15 | 38 | 28 | 91 |
| 6 | OMs | CH₂Cl₂ as solvent | -15 | 67 | 46 | 86 |
| 7 | OMs | CH₃CN as solvent | -15 | 33 | 15 | 72 |
| 8 | OMs | standard conditions | 0 | 74 | 58 | 90 |
| 9 | OMs | standard conditions | 25 | 56 | 46 | 80 |

| | | | | | | |
|---|---|---|---|---|---|---|
| ^{∗}OBz = benzoate, OTs = toluensulfonate, OMs = methansulfonate. ^{†}Deviations from standard conditions are shown. Standard conditions: PAA (27.2 mg, 0.2 mmol), BocNHOMs (84.5 mg, 0.4 mmol, 2 equiv), piperidine (82 µL, 0.8 mmol, 4 equiv), and (*R*,*R*)-FeBIP (8.9 mg, 8 mol%) in 1,2-dichlorobenzene (o-DCB, 2 mL) were stirred under N₂ atmosphere at the indicated temperature for 20 hours. ^{‡}Conversion and yields were determined by ¹H NMR analysis of the crude reaction mixture using 1,1,2,2-tetrachloroethane as an internal standard. Enantiomeric excess (ee) was determined by HPLC analysis on chiral stationary phases. ^{§}Isolated yield provided in parenthesis. | | | | | | |

Also investigated was the substrate scope for this protocol (compounds **1-43)** (Table 2). The reaction tolerated a wide range of electron-donating and -withdrawing substituents on the aromatic ring of the phenylacetic acid substrate, including methyl **(2-4),** tert-butyl **(5),** phenyl **(6),** methoxy **(7),** fluorine **(8),** chlorine **(9),** azide **(10),** 3,5-dimethyl **(11),** 1,3-dioxole **(12),** and 3,4,5-trimethoxy **(13).** The important unnatural arylglycine products **(32)** 2-13 were obtained in satisfactory yields (49-78%) and with 90-94% ee (Table 2A). Benzannulated **(14, 15)** and heteroaromatic arylglycines **(16, 17)** could also be readily prepared using this method (44-64% yield, 84-93% ee). Even more gratifyingly, the synthetic protocol also proved effective for aliphatic carboxylic acids with non-activated C(sp³)-H bonds, which represent a long-standing challenge in intermolecular asymmetric C-H amination (***14-16***) (Table 2B). The non-racemic α-amino acids **18-21,** containing primary alkyl substituents, were successfully obtained in 46-55% yield and with 85-91% ee. For sterically hindered substrates such as 2-cyclohexylacetic acid and 3,3-dimethylbutanoic acid, the desired products **22** and **23** were obtained in more modest yields of 40% and 21%, respectively, but with satisfactory 88% ee.

Next, the method according to the present inventio was applied to the synthesis of α,α-disubstituted α-amino acids, which are distinguished by their restricted conformational flexibility, increased lipophilicity and metabolic stability, as well as increased resistance toward racemization (*28, 29*) (Table 2C). Most existing synthetic routes rely on electrophilic α-alkylation of amino acid enolate equivalents. While aminations of α-branched carbonyl compounds have been achieved mainly by electrophilic hydrazinations of enolates with azidocarboxylates, the subsequent cleavage of the hydrazides to the target amines requires harsh conditions (*30-32*)*.* The method according to the present invention was applied to α-branched carboxylic acids, which would provide a general and rapid synthetic method for accessing non-racemic α,α-disubstituted α-amino acids that has to date been elusive. Indeed, when racemic α-branched carboxylic acids were reacted with BocNHOMs under iron catalysis, the tertiary α-C-H was aminated in a stereocontrolled fashion to afford the desired products in high yields and with high enantioselectivities. For example, *N*-Boc-protected α,α-disubstituted α-amino acids containing one α-methyl and one α-aryl group were synthesized in 68-89% yield and with 79-91% ee. **(24-29).** This is an enantioconvergent reaction since it starts from racemic carboxylic acids. The methyl group could be replaced with other alkyl groups such as ethyl, *n*-propyl, isopropyl, and cyclohexyl to provide the respective α,α-disubstituted α-amino acids in 59-85% yield and with 93-94% ee **(30-33).** Substituting the phenyl moiety with a thiophene provided amino acid **34** in 45% yield and with 88% ee. However, the α,α-disubstituted amino acid **35,** bearing two aliphatic substituents, was obtained in a more modest 43% yield and with 43% ee.

The method according to the present invention was also applied to the late-stage functionalization of pharmaceutically-relevant molecules (Table 2D). It was found that isoxepac **(36),** diclofenac **(37),** indomethacin **(38),** TBS-protected lithocholic acid **(39),** d-desthiobiotin **(40),** racemic ibuprofen **(41),** racemic ketoprofen **(42),** and racemic naproxen **(43)** all underwent stereocontrolled intermolecular amination in 37-88% yield and with up to 91% ee. These successful examples further underscore the utility of the one-step amino acid method according to the present invention as it enables the expedient conversion of carboxylic acid-containing drugs, drug analogs, and natural products to their respective non-racemic α-amino acids.

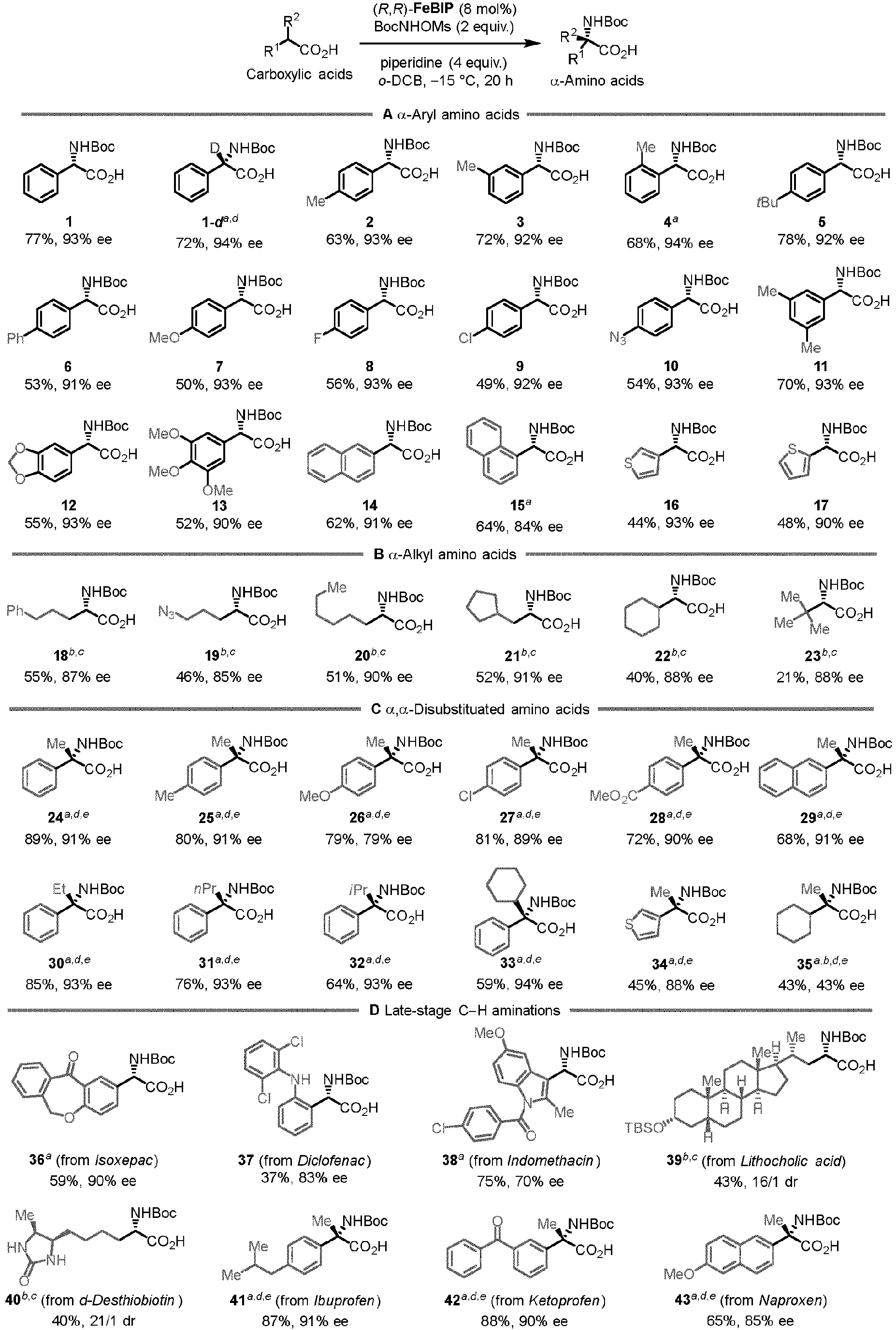

**Table 2. Direct amination scope. (A)** Scope for the enantioselective synthesis of arylglycines. **(B)** Scope for the enantioselective synthesis of α-amino acids with aliphatic side chains. **(C)** Scope for the enantioconvergent synthesis of α,α-disubstituted α-amino acids starting from racemic α-branched carboxylic acids. **(D)** Applications to the late-stage C(sp³)-H amination of drugs and natural products. *^{a}*Reaction performed with 15 mol% (*R*,*R*)-**FeBIP** for 40 hours. *^{b}*CH₂Cl₂ as the solvent instead of o-DCB. *^{c}*4 equivalents of BocNHOMs and 6 equivalents of piperidine were used instead. *^{d}*5 equivalents of BocNHOMs and 7 equivalents of piperidine were used instead. *^{e}*Reaction performed with racemic carboxylic acids.

In conclusion, it was developed an expedited catalytic asymmetric synthesis of α-amino acids by intermolecular α-C(sp³)-H amination of carboxylic acids. The method according to the present invention employs abundant and readily available carboxylic acids as starting materials and converts them in a single step into highly valuable non-racemic N-protected α-amino acids suitable for direct use in peptide synthesis and for other synthetic applications. Furthermore, the method according to the present invention utilizes the convenient Boc-protecting group and sustainable iron catalysis without the need for more toxic or less abundant transition metals. The method merges nitrene-mediated and directed C(sp³)-H functionalization without the requirement for an intermediate cyclometalation. Upon deprotonation, the carboxylic acid substrate coordinates to the iron center and serves as the directing group, which allows regioselective nitrene-mediated C(sp³)-H abstraction and ensures high stereocontrol for the subsequent C-N bond formation step. Mechanistic evidence supports a radical mechanism in which the radical rebound step, rather than the initial C(sp³)-H abstraction, is responsible for stereodiscrimination. Thus, in addition to providing a general method for the expedited synthesis of a wide range of chiral α-mono-and α,α-disubstituted amino acids, including examples of late-stage amination of carboxylic acid-containing pharmaceuticals and natural products, this study also serves as a prototype for the development of intermolecular asymmetric C-H aminations through directed C(sp³)-H nitrene insertion.

### Examples

### 1. General Information

(*R*,*R*)-**FeBIP** was prepared according to a literature procedure (19). Carboxyclic acids were obtained from commercial sources or synthesized according to standard procedures. Catalytic reactions were performed in Schlenk tubes (10 mL) under nitrogen atmosphere with magnetic stirring. Chemicals were used as received from commercial suppliers unless stated otherwise. Anhydrous CH₂Cl₂ and CH₃CN were distilled under nitrogen from calcium hydride. Anhydrous THF and Et₂O were distilled under nitrogen from sodium/benzophenone. Anhydrous 1,2-dichlorobenzene (o-DCB) was used as received from commercial suppliers. Flash column chromatography was performed with silica gel 60 M from Macherey-Nagel (230-400 mesh). ¹H and ¹³C NMR spectra were recorded on a Bruker Avance 300 MHz or a Bruker Avance 500 MHz spectrometer at ambient temperature. Chemical shifts are expressed in parts per million (δ) referenced to chloroform (7.26 ppm or 77.16 ppm), dichloromethane (5.32 ppm or 53.84 ppm) or methanol (3.31 ppm or 49.00 ppm). The NMR data are presented as follows: chemical shift, multiplicity (s = singlet, d = doublet, t = triplet, q = quartet, dd = doublet of doublet, dt = doublet of triplet, td = triplet of doublet, ddd = doublet of doublet of doublet, etc., m = multiplet, br = broad), coupling constant and integration. High resolution mass spectra (HRMS) were recorded on a Bruker En Apex Ultra 7.0 T FT-MS mass spectrometer. Optical rotations were measured using a Perkin-Elmer 241 polarimeter with [α]_{D}²⁵ values reported in degrees with concentrations reported in g/100 mL. Enantiomeric excesses (ee) and diastereomeric ratios (dr) of α-amino acids were determined by HPLC analysis using an Agilent HPLC 1260 on chiral stationary phases.

### 2. Synthesis of the Aminating Reagents

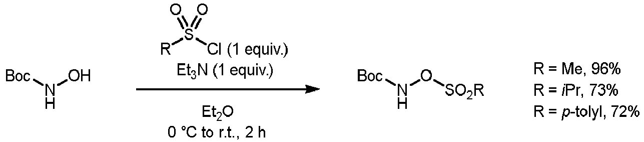

To a solution of BocNHOH (666 mg, 5 mmol, 1 equiv., received from commercial source) in diethyl ether (25 mL) at 0 °C was added Et₃N (0.70 mL, 5 mmol, 1 equiv.) and MsCl (0.39 mL, 5 mmol, 1 equiv.) dropwise. The reaction mixture was warmed to room temperature and was stirred for 2 hours. After completion, the reaction mixture was filtered, concentrated under reduced pressure, and the residue was purified by column chromatography (silica gel, eluent: EtOAc/hexane = 1/6) to afford tert-butyl ((methylsulfonyl)oxy)carbamate as a white solid (1.01 g, 96% yield): ¹H NMR (300 MHz, CDCl₃) δ 1.51 (s, 9H), 3.16 (s, 3H), 8.08 (s, 1H); ¹³C NMR (75 MHz, CDCl₃) δ 28.1, 36.4, 84.8, 154.8; HRMS calcd for [C₆H₁₃NO₅SNa]⁺ [(M + Na)⁺]: 234.0407; found: 234.0404.

Following the same procedure, tert-butyl ((isopropylsulfonyl)oxy)carbamate was prepared from BocNHOH (666 mg) and 2-propanesulfonyl chloride (0.56 mL) as a white solid (878 mg, 73% yield): ¹H NMR (300 MHz, CDCl₃) δ 1.42-1.62 (m, 15H), 3.62 (septet, *J* = 6.9 Hz, 1H), 7.85 (s, 1H); ¹³C NMR (75 MHz, CDCl₃) δ 16.7, 28.2, 51.7, 84.6, 154.7; HRMS calcd for [C₈H₁₇NO₅SNa]⁺ [(M + Na)⁺]: 262.0720; found: 262.0713.

Following the same procedure, tert-butyl (tosyloxy)carbamate was prepared from BocNHOH (666 mg) and 4-methylbenzenesulfonyl chloride (953 mg) as a white solid (1.03 g, 72% yield): ¹H NMR (300 MHz, CDCl₃) δ 1.30 (s, 9H), 2.46 (s, 3H), 7.31-7.43 (m, 2H), 7.71 (s, 1H), 7.82-7.95 (m, 2H); ¹³C NMR (75 MHz, CDCl₃) δ 21.8, 27.8, 84.0, 129.76, 129.80, 130.8, 146.0, 154.3; HRMS calcd for [C₁₂H₁₇NO₅SNa]⁺ [(M + Na)⁺]: 310.0720; found: 310.0713.

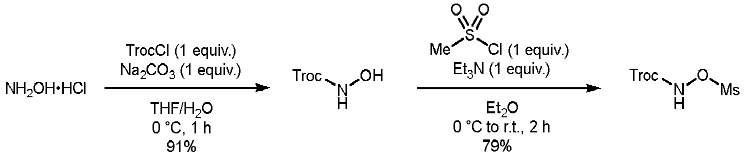

To a solution of hydroxylamine hydrochloride (2.08 g, 30 mmol, 1 equiv.) and Na₂CO₃ (3.18 g, 30 mmol, 1 equiv.) in tetrahydrofuran/water (v/v = 1/1, 60 mL) at 0 °C was added 2,2,2-trichloroethyl chloroformate (4.1 mL, 30 mmol, 1 equiv.) dropwise. The reaction mixture was stirred at 0 °C for 1 hour. After completion, the reaction mixture was extracted with EtOAc for three times. The combined organic layer was washed with brine and was dried over anhydrous sodium sulfate. After filtration, the solvent was evaporated under reduced pressure, and the residue was purified by column chromatography (silica gel, eluent: EtOAc/hexane = 1/2) to afford 2,2,2-trichloroethyl hydroxycarbamate as a white solid (5.69 g, 91% yield).

To a solution of 2,2,2-trichloroethyl hydroxycarbamate (417 mg, 2 mmol, 1 equiv.) in diethyl ether (10 mL) at 0 °C was added Et₃N (0.28 mL, 2 mmol, 1 equiv.) and MsCl (0.15 mL, 2 mmol, 1 equiv.) dropwise. The reaction mixture was warmed to room temperature and was stirred for 2 hours. After completion, the reaction mixture was filtered, concentrated under reduced pressure, and the residue was purified by column chromatography (silica gel, eluent: EtOAc/hexane = 1/3) to afford 2,2,2-trichloroethyl ((methylsulfonyl)oxy)carbamate as a white solid (451 mg, 79% yield): ¹H NMR (300 MHz, CDCl₃) δ 3.23 (s, 3H), 4.86 (s, 2H), 8.72 (s, 1H); ¹³C NMR (75 MHz, CDCl₃) δ 36.7, 75.6, 94.2, 154.4; HRMS calcd for [C₄H₆Cl₃NO₅SNa]⁺ [(M + Na)⁺]: 307.8924; found: 307.8924.

### 3. Iron Catalyzed Enantioselective α-Amination of Carboxylic Acids

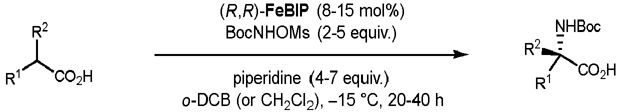

**General procedure for the iron catalyzed α-amination of carboxylic acids:** to a dry Schlenk tube was added the carboxylic acid (0.2 mmol, 1 equiv.), BocNHOMs (2-5 equiv.) and (*R*,*R*)-**FeBIP** (8-15 mol%). The tube was evacuated and backfilled with N₂ for five times. The indicated solvent (2 mL, 0.1 M) and piperidine (4-7 equiv.) were added via syringe, and the tube was sealed. The reaction mixture was further degassed via freeze-pump-thaw for one time. Then the reaction mixture was stirred at -15 °C for the indicated time. After completion, the reaction mixture was diluted with 30 mL Et₂O and was washed with aqueous NaHSO₄ (1 M) for two times (10 mL for each time) for removing the majority of iron complex from the Et₂O layer. The combined aqueous layer was extracted with Et₂O (20×3 mL). The combined organic layer was dried over anhydrous sodium sulfate. After filtration, the solvent was evaporated under reduced pressure, and the residue was purified by column chromatography on silica gel using indicated solvent as the eluent (Note: thin-layer chromatography was conducted in a mixed solvent of MeOH/CH₂Cl₂ = 1/4 with 0.5% NH₃•H₂O, in which the *in situ* formed ammonium salt of *N*-Boc-protected α-amino acid is always less polar than the ammonium salt of the starting carboxylic acid).

### (S)-2-((tert-Butoxycarbonyl)amino)-2-phenylacetic acid (1)

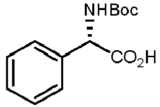

Following the **general procedure** [(*R*,*R*)-**FeBIP** (8 mol%), BocNHOMs (2 equiv.) and piperidine (4 equiv.) in o-DCB for 20 h], the α-amination of 2-phenylacetic acid (27.2 mg, 0.2 mmol) gave **1** as a colorless foam in 77% yield (eluent: EtOAc/hexane = 1/4 with 0.2% HOAc, 38.6 mg) with 93% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 14.7 min, t₂ = 21.7 min]: [α]_{D}²⁵ = +117.6 (c 1.0, MeOH, 93% ee); ¹H NMR (300 MHz, CD₃OD) δ 1.33-1.51 (br, 9H), 5.00-5.27 (br, 1H), 7.23-7.47 (m, 5H); ¹³C NMR (75 MHz, CD₃OD) δ 28.7, 59.2, 80.8, 128.5, 129.2, 129.7, 138.7, 157.4, 174.2; HRMS calcd for [C₁₃H₁₇NO₄Na]⁺ [(M + Na)⁺]: 274.1050; found: 274.1045.

### (S)-2-((tert-Butoxycarbonyl)amino)-2-phenylacetic-2-d acid (1-d)

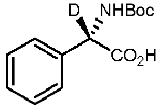

Following the **general procedure** [(*R*,*R*)-**FeBIP** (15 mol%), BocNHOMs (5 equiv.) and piperidine (7 equiv.) in o-DCB for 40 h], the α-amination of 2-phenylacetic-2,2-*d*₂ acid (27.6 mg, 0.2 mmol) gave **1-*d*** as a colorless foam in 72% yield (eluent: EtOAc/hexane = 1/4 with 0.2% HOAc, 36.6 mg) with 94% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 14.4 min, t₂ = 21.1 min]: [α]_{D}²⁵ = +118.6 (c 1.0, MeOH, 94% ee); ¹H NMR (500 MHz, CD₃OD) δ 1.34-1.47 (br, 9H), 7.28-7.38 (m, 3H), 7.38-7.43 (m, 2H); ¹³C NMR (125 MHz, CD₃OD) δ 28.7, 58.9 (t, *J* = 21.5 Hz), 80.8, 128.5, 129.2, 129.7, 138.6, 157.5, 174.2; HRMS calcd for [C₁₃H₁₆DNO₄Na]⁺ [(M + Na)⁺]: 275.1113; found: 275.1105.

### (S)-2-((tert-Butoxycarbonyl)amino)-2-(p-tolyl)acetic acid (2)

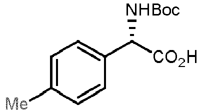

Following the **general procedure** [(*R*,*R*)-**FeBIP** (8 mol%), BocNHOMs (2 equiv.) and piperidine (4 equiv.) in o-DCB for 20 h], the α-amination of 2-(p-tolyl)acetic acid (30.0 mg, 0.2 mmol) gave 2 as a colorless foam in 63% yield (eluent: EtOAc/hexane = 1/5 with 0.2% HOAc, 33.5 mg) with 93% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 16.3 min, t₂ = 20.3 min]: [α]_{D}²⁵ = +134.4 (c 1.0, MeOH, 93% ee); ¹H NMR (300 MHz, CD₃OD) δ 1.28-1.57 (br, 9H), 2.32 (s, 3H), 4.97-5.25 (br, 1H), 7.08-7.21 (m, 2H), 7.22-7.33 (m, 2H); ¹³C NMR (75 MHz, CD₃OD) δ 21.1, 28.7, 58.9, 80.8, 128.4, 130.3, 135.6, 139.2, 157.5, 174.4; HRMS calcd for [C₁₄H₁₉NO₄Na]⁺ [(M + Na)⁺]: 288.1206; found: 288.1202.

### (S)-2-((tert-Butoxycarbonyl)amino)-2-(m-tolyl)acetic acid (3)

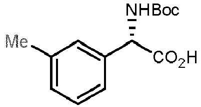

Following the **general procedure** [(*R*,*R*)-**FeBIP** (8 mol%), BocNHOMs (2 equiv.) and piperidine (4 equiv.) in o-DCB for 20 h], the α-amination of 2-(*m*-tolyl)acetic acid (30.0 mg, 0.2 mmol) gave 3 as a colorless oil in 72% yield (eluent: EtOAc/hexane = 1/5 with 0.2% HOAc, 38.3 mg) with 92% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 13.0 min, t₂ = 17.8 min]: [α]_{D}²⁵ = +122.2 (c 1.0, MeOH, 92% ee); ¹H NMR (300 MHz, CD₃OD) δ 1.29-1.58 (br, 9H), 2.33 (s, 3H), 4.96-5.25 (br, 1H), 7.06-7.32 (m, 4H); ¹³C NMR (75 MHz, CD₃OD) δ 21.4, 28.7, 59.2, 80.8, 125.6, 129.2, 129.6, 129.9, 138.6, 139.5, 157.4, 174.3; HRMS calcd for [C₁₄H₁₉NO₄Na]⁺ [(M + Na)⁺]: 288.1206; found: 288.1202.

### (S)-2-((tert-Butoxycarbonyl)amino)-2-(o-tolyl)acetic acid (4)

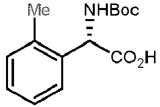

Following the **general procedure** [(*R*,*R*)-**FeBIP** (15 mol%), BocNHOMs (2 equiv.) and piperidine (4 equiv.) in o-DCB for 40 h], the α-amination of 2-(*o-*tolyl)acetic acid (30.0 mg, 0.2 mmol) gave **4** as a colorless oil in 68% yield (eluent: EtOAc/hexane = 1/5 with 0.2% HOAc, 36.3 mg) with 94% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 13.2 min, t₂ = 17.6 min]: [α]_{D}²⁵ = +126.2 (c 1.0, MeOH, 94% ee); ¹H NMR (300 MHz, CD₃OD) δ 1.30-1.54 (br, 9H), 2.45 (s, 3H), 5.25-5.59 (br, 1H), 7.11-7.23 (m, 3H), 7.23-7.34 (m, 1H); ¹³C NMR (75 MHz, CD₃OD) δ 19.5, 28.7, 55.7, 80.7, 127.3, 127.8, 129.2, 131.6, 137.1, 137.8, 157.6, 174.7; HRMS calcd for [C₁₄H₁₉NO₄Na]⁺ [(M + Na)⁺]: 288.1206; found: 288.1201.

### (S)-2-((tert-Butoxycarbonyl)amino)-2-(4-(tert-butyl)phenyl)acetic acid (5)

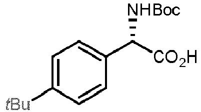

Following the **general procedure** [(*R*,*R*)-**FeBIP** (8 mol%), BocNHOMs (2 equiv.) and piperidine (4 equiv.) in o-DCB for 20 h], the α-amination of 2-(4-(*tert*-butyl)phenyl)acetic acid (38.5 mg, 0.2 mmol) gave 5 as a colorless oil in 78% yield (eluent: EtOAc/hexane = 1/5 with 0.2% HOAc, 48.1 mg) with 92% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 12.3 min, t₂ = 17.5 min]: [α]_{D}²⁵ =+117.0 (c 1.0, MeOH, 92% ee); ¹H NMR (300 MHz, CD₃OD) δ 1.31 (s, 9H), 1.35-1.51 (br, 9H), 4.99-5.25 (br, 1H), 7.26-7.36 (m, 2H), 7.36-7.47 (m, 2H); ¹³C NMR (75 MHz, CD₃OD) δ 28.7, 31.7, 35.4, 58.8, 80.8, 126.6, 128.2, 135.5, 152.4, 157.4, 174.4; HRMS calcd for [C₁₇H₂₅NO₄Na]⁺ [(M + Na)⁺]: 330.1676; found: 330.1673.

### (S)-2-([1,1'-Biphenyl]-4-yl)-2-((tert-butoxycarbonyl)amino)acetic acid (6)

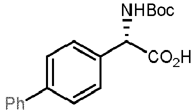

Following the **general procedure** [(*R*,*R*)-**FeBIP** (8 mol%), BocNHOMs (2 equiv.) and piperidine (4 equiv.) in o-DCB for 20 h], the α-amination of 2-([1,1'-biphenyl]-4-yl)acetic acid (42.4 mg, 0.2 mmol) gave 6 as a colorless foam in 53% yield (eluent: EtOAc/hexane = 1/4 with 0.2% HOAc, 34.9 mg) with 91% ee [DAICEL CHIRALCEL OD-H column, Agilent HPLC 1260, *i*PrOH/hexane = 10/90 (v/v) with 0.1% TFA, 1.0 mL/min, 25 °C, 210 nm; t₁ = 5.9 min, t₂ = 6.7 min]: [α]_{D}²⁵ = +143.5 (c 1.0, MeOH, 91% ee); ¹H NMR (300 MHz, CD₃OD) δ 1.33-1.56 (br, 9H), 5.03-5.38 (br, 1H), 7.27-7.36 (m, 1H), 7.37-7.45 (m, 2H), 7.45-7.52 (m, 2H), 7.54-7.68 (m, 4H); ¹³C NMR (75 MHz, CD₃OD) δ 28.7, 58.9, 80.8, 128.0, 128.2, 128.5, 129.0, 129.9, 137.7, 141.8, 142.4, 157.5, 174.1; HRMS calcd for [C₁₉H₂₁NO₄Na]⁺ [(M + Na)⁺]: 350.1363; found: 350.1358.

### (S)-2-((tert-Butoxycarbonyl)amino)-2-(4-methoxyphenyl)acetic acid (7)

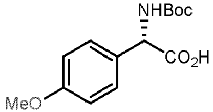

Following the **general procedure** [(*R*,*R*)-**FeBIP** (8 mol%), BocNHOMs (2 equiv.) and piperidine (4 equiv.) in o-DCB for 20 h], the α-amination of 2-(4-methoxyphenyl)acetic acid (33.2 mg, 0.2 mmol) gave 7 as a colorless foam in 50% yield (eluent: EtOAc/hexane = 1/3 with 0.2% HOAc, 28.2 mg) with 93% ee [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 11.7 min, t₂ = 15.2 min]: [α]_{D}²⁵ = +138.4 (c 1.0, MeOH, 93% ee); ¹H NMR (300 MHz, CD₃OD) δ 1.33-1.50 (br, 9H), 3.78 (s, 3H), 4.96-5.21 (br, 1H), 6.83-6.97 (m, 2H), 7.24-7.39 (m, 2H); ¹³C NMR (75 MHz, CD₃OD) δ 28.7, 55.7, 58.6, 80.8, 115.1, 129.8, 130.5, 157.5, 161.1, 174.6; HRMS calcd for [C₁₄H₁₉NO₅Na]⁺ [(M + Na)⁺]: 304.1155; found: 304.1152.

### (S)-2-((tert-Butoxycarbonyl)amino)-2-(4-fluorophenyl)acetic acid (8)

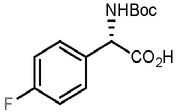

Following the **general procedure** [(*R*,*R*)-**FeBIP** (8 mol%), BocNHOMs (2 equiv.) and piperidine (4 equiv.) in o-DCB for 20 h], the α-amination of 2-(4-fluorophenyl)acetic acid (30.8 mg, 0.2 mmol) gave **8** as a colorless foam in 56% yield (eluent: EtOAc/hexane = 1/4 with 0.2% HOAc, 30.3 mg) with 93% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 11.1 min, t₂ = 16.9 min]: [α]_{D}²⁵ = +121.1 (c 1.0, MeOH, 93% ee); ¹H NMR (300 MHz, CD₃OD) δ 1.32-1.53 (br, 9H), 5.00-5.29 (br, 1H), 7.00-7.17 (m, 2H), 7.32-7.52 (m, 2H); ¹³C NMR (75 MHz, CD₃OD) δ 28.7, 58.5, 80.9, 116.3 (d, *J* = 21.9 Hz), 130.5 (d, *J* = 8.3 Hz), 134.9 (d, *J* = 2.2 Hz), 157.4, 164.0 (d, *J* = 245.3 Hz), 174.0; HRMS calcd for [C₁₃H₁₆FNO₄Na]⁺ [(M + Na)⁺]: 292.0956; found: 292.0951.

### (S)-2-((tert-Butoxycarbonyl)amino)-2-(4-chlorophenyl)acetic acid (9)

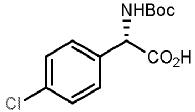

Following the **general procedure** [(*R*,*R*)-**FeBIP** (8 mol%), BocNHOMs (2 equiv.) and piperidine (4 equiv.) in o-DCB for 20 h], the α-amination of 2-(4-chlorophenyl)acetic acid (34.1 mg, 0.2 mmol) gave 9 as a colorless foam in 49% yield (eluent: EtOAc/hexane = 1/4 with 0.2% HOAc, 27.8 mg) with 92% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 11.4 min, t₂ = 16.3 min]: [α]_{D}²⁵ = +133.8 (c 1.0, MeOH, 92% ee); ¹H NMR (300 MHz, CD₃OD) δ 1.32-1.53 (br, 9H), 5.01-5.30 (br, 1H), 7.28-7.46 (m, 4H); ¹³C NMR (75 MHz, CD₃OD) δ 28.7, 58.5, 80.9, 129.7, 130.2, 135.0, 137.7, 157.4, 173.6; HRMS calcd for [C₁₃H₁₆ClNO₄Na]⁺ [(M + Na)⁺]: 308.0660; found: 308.0657.

### (S)-2-(4-Azidophenyl)-2-((tert-butoxycarbonyl)amino)acetic acid (10)

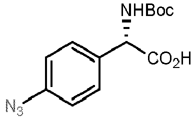

Following the **general procedure** [(*R*,*R*)-**FeBIP** (8 mol%), BocNHOMs (2 equiv.) and piperidine (4 equiv.) in o-DCB for 20 h], the α-amination of 2-(4-azidophenyl)acetic acid (35.4 mg, 0.2 mmol) gave **10** as a light-yellow foam in 54% yield (eluent: EtOAc/hexane = 1/4 with 0.2% HOAc, 31.4 mg) with 93% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 18.9 min, t₂ = 23.9 min]: [α]_{D}²⁵ = +146.9 (c 1.0, MeOH, 93% ee); ¹H NMR (300 MHz, CD₃OD) δ 1.34-1.55 (br, 9H), 5.00-5.28 (br, 1H), 6.97-7.15 (m, 2H), 7.32-7.54 (m, 2H); ¹³C NMR (75 MHz, CD₃OD) δ 28.7, 58.6, 80.9, 120.2, 130.2, 135.6, 141.4, 157.4, 173.9; HRMS calcd for [C₁₃H₁₆N₄O₄Na]⁺ [(M + Na)⁺]: 315.1064; found: 315.1064.

### (S)-2-((tert-Butoxycarbonyl)amino)-2-(3,5-dimethylphenyl)acetic acid (11)

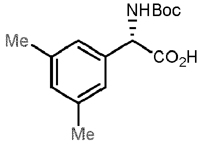

Following the **general procedure** [(*R*,*R*)-**FeBIP** (8 mol%), BocNHOMs (2 equiv.) and piperidine (4 equiv.) in o-DCB for 20 h], the α-amination of 2-(3,5-dimethylphenyl)acetic acid (32.8 mg, 0.2 mmol) gave **11** as a colorless foam in 70% yield (eluent: EtOAc/hexane = 1/5 with 0.2% HOAc, 39.2 mg) with 93% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 11.3 min, t₂ = 15.0 min]: [α]_{D}²⁵ = +126.4 (c 1.0, MeOH, 93% ee); ¹H NMR (300 MHz, CD₃OD) δ 1.32-1.54 (br, 9H), 2.29 (s, 6H), 4.94-5.18 (br, 1H), 6.90-6.97 (m, 1H), 6.97-7.07 (m, 2H); ¹³C NMR (75 MHz, CD₃OD) δ 21.3, 28.7, 59.1, 80.8, 126.3, 130.7, 138.4, 139.4, 157.4, 174.4; HRMS calcd for [C₁₅H₂₁NO₄Na]⁺ [(M + Na)⁺]: 302.1363; found: 302.1355.

### (S)-2-(Benzo[d][1,3]dioxol-5-yl)-2-((tert-butoxycarbonyl)amino)acetic acid (12)

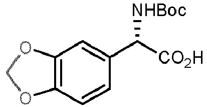

Following the **general procedure** [(*R*,*R*)-**FeBIP** (8 mol%), BocNHOMs (2 equiv.) and piperidine (4 equiv.) in o-DCB for 20 h], the α-amination of 2-(benzo[*d*][1,3]dioxol-5-yl)acetic acid (36.0 mg, 0.2 mmol) gave **12** as a colorless foam in 55% yield (eluent: EtOAc/hexane = 1/3 with 0.2% HOAc, 32.8 mg) with 93% ee [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 9.9 min, t₂ = 12.5 min]: [α]_{D}²⁵ = +135.0 (c 1.0, MeOH, 93% ee); ¹H NMR (300 MHz, CD₃OD) δ 1.30-1.60 (br, 9H), 4.94-5.20 (br, 1H), 5.93 (s, 2H), 6.72-6.82 (m, 1H), 6.82-6.94 (m, 2H); ¹³C NMR (75 MHz, CD₃OD) δ 28.7, 58.9, 80.8, 102.6, 108.8, 109.2, 122.2, 132.3, 149.0, 149.3, 157.4, 174.2; HRMS calcd for [C₁₄H₁₇NO₆Na]⁺ [(M + Na)⁺]: 318.0948; found: 318.0939.

### (S)-2-((tert-Butoxycarbonyl)amino)-2-(3,4,5-trimethoxyphenyl)acetic acid (13)

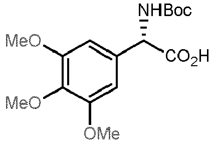

Following the **general procedure** [(*R*,*R*)-**FeBIP** (8 mol%), BocNHOMs (2 equiv.) and piperidine (4 equiv.) in o-DCB for 20 h], the α-amination of 2-(3,4,5-trimethoxyphenyl)acetic acid (45.2 mg, 0.2 mmol) gave **13** as a colorless foam in 52% yield (eluent: EtOAc/hexane = 1/1 with 0.2% HOAc, 35.6 mg) with 90% ee [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 10.9 min, t₂ = 15.6 min]: [α]_{D}²⁵ = +121.2 (c 1.0, MeOH, 90% ee); ¹H NMR (300 MHz, CD₃OD) δ 1.27-1.59 (br, 9H), 3.75 (s, 3H), 3.83 (s, 6H), 4.97-5.21 (br, 1H), 6.73 (s, 2H); ¹³C NMR (75 MHz, CD₃OD) δ 28.7, 56.6, 59.3, 61.1, 80.8, 106.0, 134.5, 139.0, 154.6, 157.5, 174.2; HRMS calcd for [C₁₆H₂₃NO₇Na]⁺ [(M + Na)⁺]: 364.1367; found: 364.1360.

### (S)-2-((tert-Butoxycarbonyl)amino)-2-(naphthalen-2-yl)acetic acid (14)

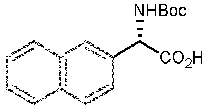

Following the **general procedure** [(*R*,*R*)-**FeBIP** (8 mol%), BocNHOMs (2 equiv.) and piperidine (4 equiv.) in o-DCB for 20 h], the α-amination of 2-(naphthalen-2-yl)acetic acid (37.2 mg, 0.2 mmol) gave **14** as a colorless foam in 62% yield (eluent: EtOAc/hexane = 1/4 with 0.2% HOAc, 37.5 mg) with 91% ee [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 9.0 min, t₂ = 11.4 min]: [α]_{D}²⁵ = +152.5 (c 1.0, MeOH, 91% ee); ¹H NMR (300 MHz, CD₃OD) δ 1.31-1.56 (br, 9H), 5.16-5.54 (br, 1H), 7.38-7.59 (m, 3H), 7.73-7.95 (m, 4H); ¹³C NMR (75 MHz, CD₃OD) δ 28.7, 59.3, 80.9, 126.2, 127.3, 127.4, 127.6, 128.6, 129.0, 129.4, 134.5, 134.7, 136.0, 157.5, 174.1; HRMS calcd for [C₁₇H₁₉NO₄Na]⁺ [(M + Na)⁺]: 324.1206; found: 324.1203.

### (S)-2-((tert-Butoxycarbonyl)amino)-2-(naphthalen-1-yl)acetic acid (15)

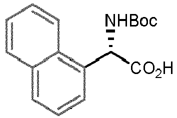

Following the **general procedure** [(*R*,*R*)-**FeBIP** (15 mol%), BocNHOMs (2 equiv.) and piperidine (4 equiv.) in o-DCB for 40 h], the α-amination of 2-(naphthalen-1-yl)acetic acid (37.2 mg, 0.2 mmol) gave **15** as a colorless foam in 64% yield (eluent: EtOAc/hexane = 1/4 with 0.2% HOAc, 38.7 mg) with 84% ee [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 8.7 min, t₂ = 11.0 min]: [α]_{D}²⁵ = +128.1 (c 1.0, MeOH, 84% ee); ¹H NMR (300 MHz, CD₃OD) δ 1.20-1.56 (br, 9H), 5.71-6.15 (br, 1H), 7.19-7.66 (m, 4H), 7.66-8.00 (m, 2H), 8.00-8.34 (m, 1H); ¹³C NMR (75 MHz, CD₃OD) δ 28.7, 55.9, 80.8, 124.5, 126.3, 126.6, 127.0, 127.6, 129.8, 130.0, 132.6, 134.6, 135.5, 157.6, 174.8; HRMS calcd for [C₁₇H₁₉NO₄Na]⁺ [(M + Na)⁺]: 324.1206; found: 324.1199.

### (S)-2-((tert-Butoxycarbonyl)amino)-2-(thiophen-3-yl)acetic acid (16)

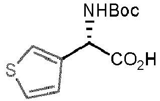

Following the **general procedure** [(*R*,*R*)-**FeBIP** (8 mol%), BocNHOMs (2 equiv.) and piperidine (4 equiv.) in o-DCB for 20 h], the α-amination of 2-(thiophen-3-yl)acetic acid (28.4 mg, 0.2 mmol) gave **16** as a light-yellow oil in 44% yield (eluent: EtOAc/hexane = 1/4 with 0.2% HOAc, 22.6 mg) with 93% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 18.7 min, t₂ = 21.2 min]: [α]_{D}²⁵ = +108.1 (c 1.0, MeOH, 93% ee); ¹H NMR (300 MHz, CD₃OD) δ 1.49 (s, 9H), 5.11-5.46 (br, 1H), 7.09-7.19 (m, 1H), 7.32-7.48 (m, 2H); ¹³C NMR (75 MHz, CD₃OD) δ 28.7, 55.0, 80.8, 123.9, 127.2, 127.8, 138.6, 157.6, 174.0; HRMS calcd for [C₁₁H₁₅NO₄SNa]⁺ [(M + Na)⁺]: 280.0614; found: 280.0612.

### (S)-2-((tert-Butoxycarbonyl)amino)-2-(thiophen-2-yl)acetic acid (17)

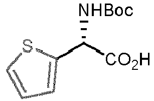

Following the **general procedure** [(*R*,*R*)-**FeBIP** (8 mol%), BocNHOMs (2 equiv.) and piperidine (4 equiv.) in o-DCB for 20 h], the α-amination of 2-(thiophen-2-yl)acetic acid (28.4 mg, 0.2 mmol) gave **17** as a light-yellow oil in 48% yield (eluent: EtOAc/hexane = 1/4 with 0.2% HOAc, 24.8 mg) with 90% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 16.1 min, t₂ = 20.7 min]: [α]_{D}²⁵ = +105.3 (c 1.0, MeOH, 90% ee); ¹H NMR (300 MHz, CD₃OD) δ 1.45 (s, 9H), 5.25-5.57 (br, 1H), 6.98 (dd, *J=* 5.0, 3.8 Hz, 1H), 7.03-7.18 (m, 1H), 7.27-7.46 (m, 1H); ¹³C NMR (75 MHz, CD₃OD) δ 28.7, 54.7, 81.0, 126.5, 127.1, 127.8, 140.8, 157.4, 173.2; HRMS calcd for [C₁₁H₁₅NO₄SNa]⁺ [(M + Na)⁺]: 280.0614; found: 280.0608.

### (S)-2-((tert-Butoxycarbonyl)amino)-5-phenylpentanoic acid (18)

Following the **general procedures** [(*R*,*R*)-**FeBIP** (8 mol%), BocNHOMs (4 equiv.) and 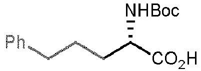 piperidine (6 equiv.) in CH₂Cl₂ for 20 h], the α-amination of 5-phenylpentanoic acid (35.6 mg, 0.2 mmol) gave **18** as a colorless oil in 55% yield (eluent: EtOAc/hexane = 1/5 with 0.2% HOAc, 32.3 mg) with 87% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 13.9 min, t₂ = 20.6 min]: [α]_{D}²⁵ = +11.7 (c 1.0, MeOH, 87% ee); ¹H NMR (300 MHz, CD₃OD) δ 1.44 (s, 9H), 1.58-1.76 (m, 3H), 1.76-1.91 (m, 1H), 2.52-2.73 (m, 2H), 3.81-4.23 (br, 1H), 7.09-7.20 (m, 3H), 7.20-7.32 (m, 2H); ¹³C NMR (75 MHz, CD₃OD) δ 28.7, 28.8, 32.4, 36.2, 54.6, 80.4, 126.8, 129.3, 129.4, 143.2, 158.1, 176.2; HRMS calcd for [C₁₆H₂₄NO₄]⁺ [(M + H)⁺]: 294.1700; found: 294.1703.

### (S)-5-Azido-2-((tert-butoxycarbonyl)amino)pentanoic acid (19)

Following the **general procedure** [(*R*,*R*)-**FeBIP** (8 mol%), BocNHOMs (4 equiv.) and 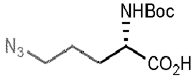 piperidine (6 equiv.) in CH₂Cl₂ for 20 h], the α-amination of 5-azidopentanoic acid (28.6 mg, 0.2 mmol) gave **19** as a colorless oil in 46% yield (eluent: EtOAc/hexane = 1/4 with 0.2% HOAc, 23.8 mg) with 85% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 12.8 min, t₂ = 19.6 min]: [α]_{D}²⁵ = +19.5 (c 0.5, MeOH, 85% ee); ¹H NMR (300 MHz, CD₃OD) δ 1.45 (s, 9H), 1.59-1.78 (m, 3H), 1.80-2.01 (m, 1H), 3.30-3.39 (m, 2H), 3.92-4.20 (br, 1H); ¹³C NMR (75 MHz, CD₃OD) δ 26.4, 28.7, 30.1, 52.0, 54.4, 80.6, 158.1, 175.8; HRMS calcd for [C₁₀H₁₈N₄O₄Na]⁺ [(M + Na)⁺]: 281.1220; found: 281.1217.

### (S)-2-((tert-Butoxycarbonyl)amino)octanoic acid (20)

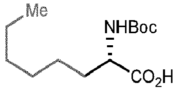

Following the **general procedure** [(*R*,*R*)-**FeBIP** (8 mol%), BocNHOMs (4 equiv.) and piperidine (6 equiv.) in CH₂Cl₂ for 20 h], the α-amination of octanoic acid (28.8 mg, 0.2 mmol) gave **20** as a colorless oil in 51% yield (eluent: EtOAc/hexane = 1/6 with 0.2% HOAc, 26.7 mg) with 90% ee [DAICEL CHIRALPAK IG column, Agilent HPLC 1260, *i*PrOH/hexane = 5/95 (v/v) with 0.1% TFA, 1.0 mL/min, 25 °C, 210 nm; t₁ = 21.1 min, t₂ = 29.2 min]: [α]_{D}²⁵ = +4.5 (c 1.0, MeOH, 90% ee); ¹H NMR (300 MHz, CD₃OD) δ 0.86-0.96 (m, 3H), 1.23-1.41 (m, 8H), 1.44 (s, 9H), 1.55-1.68 (m, 1H), 1.68-1.88 (m, 1H), 3.90-4.16 (br, 1H); ¹³C NMR (75 MHz, CD₃OD) δ 14.4, 23.6, 26.8, 28.7, 29.9, 32.8, 32.9, 54.8, 80.4, 158.1, 176.4; HRMS calcd for [C₁₃H₂₅NO₄Na]⁺ [(M + Na)⁺]: 282.1676; found: 282.1672.

### (S)-2-((tert-Butoxycarbonyl)amino)-3-cyclopentylpropanoic acid (21)

Following the **general procedure** [(*R*,*R*)-**FeBIP** (8 mol%), BocNHOMs (4 equiv.) and 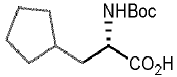 piperidine (6 equiv.) in CH₂Cl₂ for 20 h], the α-amination of 3-cyclopentylpropanoic acid (28.4 mg, 0.2 mmol) gave **21** as a colorless oil in 52% yield (eluent: EtOAc/hexane = 1/6 with 0.2% HOAc, 26.7 mg) with 91% ee [DAICEL CHIRALPAK IA column, Agilent HPLC 1260, *i*PrOH/hexane = 10/90 (v/v) with 0.1% TFA, 1.0 mL/min, 25 °C, 210 nm; t₁ = 8.0 min, t₂ = 14.1 min]: [α]_{D}²⁵ = +16.7 (c 0.5, MeOH, 91% ee); ¹H NMR (300 MHz, CD₃OD) δ 1.05-1.22 (m, 2H), 1.44 (s, 9H), 1.54-1.75 (m, 6H), 1.77-1.88 (m, 2H), 1.88-2.03 (m, 1H), 3.90-4.17 (br, 1H); ¹³C NMR (75 MHz, CD₃OD) δ 25.9, 26.1, 28.7, 33.2, 33.8, 38.2, 39.1, 54.6, 80.4, 158.1, 176.7; HRMS calcd for [C₁₃H₂₃NO₄Na]⁺ [(M + Na)⁺]: 280.1519; found: 280.1510.

### (S)-2-((tert-Butoxycarbonyl)amino)-2-cyclohexylacetic acid (22)

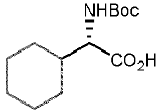

Following the **general procedure** [(*R*,*R*)-**FeBIP** (8 mol%), BocNHOMs (4 equiv.) and piperidine (6 equiv.) in CH₂Cl₂ for 20 h], the α-amination of 2-cyclohexylacetic acid (28.4 mg, 0.2 mmol) gave **22** as a colorless oil in 40% yield (eluent: EtOAc/hexane = 1/6 with 0.2% HOAc, 20.5 mg) with 88% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 11.3 min, t₂ = 21.4 min]: [α]_{D}²⁵ = +22.7 (c 0.5, MeOH, 88% ee); ¹H NMR (300 MHz, CD₃OD) δ 1.08-1.34 (m, 5H), 1.44 (s, 9H), 1.60-1.84 (m, 6H), 3.78-4.12 (br, 1H); ¹³CNMR (75 MHz, CD₃OD) δ 27.16, 27.19 (2C), 28.7, 29.4, 30.8, 41.5, 60.0, 80.5, 158.2, 175.5; HRMS calcd for [C₁₃H₂₃NO₄Na]⁺ [(M + Na)⁺]: 280.1519; found: 280.1515.

### (S)-2-((tert-Butoxycarbonyl)amino)-3,3-dimethylbutanoic acid (23)

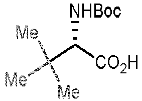

Following the **general procedure** [(*R*,*R*)-**FeBIP** (8 mol%), BocNHOMs (4 equiv.) and piperidine (6 equiv.) in CH₂Cl₂ for 20 h], the α-amination of 3,3-dimethylbutanoic acid (46.5 mg, 0.4 mmol) gave **23** as a colorless foam in 21% yield (eluent: EtOAc/hexane = 1/6 with 0.2% HOAc, 19.8 mg) with 88% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 9.7 min, t₂ = 13.8 min]: [α]_{D}²⁵ = +23.6 (c 0.5, MeOH, 88% ee); ¹H NMR (300 MHz, CD₃OD) δ 1.00 (s, 9H), 1.45 (s, 9H), 3.78-4.01 (br, 1H); ¹³C NMR (75 MHz, CD₃OD) δ 27.1, 28.7, 34.9, 63.5, 80.6, 158.0, 174.9; HRMS calcd for [C₁₁H₂₁NO₄Na]⁺ [(M + Na)⁺]: 254.1363; found: 254.1361.

### (S)-2-((tert-Butoxycarbonyl)amino)-2-phenylpropanoic acid (24)

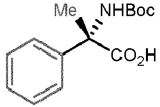

Following the **general procedure** [(*R*,*R*)-**FeBIP** (15 mol%), BocNHOMs (5 equiv.) and piperidine (7 equiv.) in o-DCB for 40 h], the α-amination of (±)-2-phenylpropanoic acid (30.0 mg, 0.2 mmol) gave **24** as a colorless foam in 89% yield (eluent: EtOAc/hexane = 1/6 with 0.2% HOAc, 47.4 mg) with 91% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 7.9 min, t₂ = 13.2 min]: [α]_{D}²⁵ = +51.1 (c 1.0, MeOH, 91% ee); ¹H NMR (300 MHz, CD₃OD) δ 1.16-1.51 (br, 9H), 1.92 (s, 3H), 7.20-7.42 (m, 3H), 7.42-7.57 (m, 2H); ¹³C NMR (75 MHz, CD₃OD) δ 24.1, 28.6, 62.8, 80.8, 127.1, 128.6, 129.3, 142.8, 156.6, 176.1; HRMS calcd for [C₁₄H₂₀NO₄]+ [(M + H)⁺]: 266.1387; found: 266.1383.

### (S)-2-((tert-Butoxycarbonyl)amino)-2-(p-tolyl)propanoic acid (25)

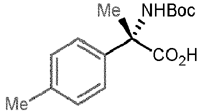

Following the **general procedure** [(*R*,*R*)-**FeBIP** (15 mol%), BocNHOMs (5 equiv.) and piperidine (7 equiv.) in o-DCB for 40 h], the α-amination of (±)-2-(*p*-tolyl)propanoic acid (32.8 mg, 0.2 mmol) gave **25** as a colorless oil in 80% yield (eluent: EtOAc/hexane = 1/5 with 0.2% HOAc, 44.6 mg) with 91% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 7.9 min, t₂ = 11.8 min]: [α]_{D}²⁵ = +46.8 (c 1.0, MeOH, 91% ee); ¹H NMR (300 MHz, CD₃OD) δ 1.07-1.58 (br, 9H), 1.89 (s, 3H), 2.31 (s, 3H), 7.06-7.24 (m, 2H), 7.26-7.50 (m, 2H); ¹³C NMR (75 MHz, CD₃OD) δ 21.0, 24.1, 28.6, 62.6, 80.7, 127.0, 129.9, 138.4, 139.8, 156.6, 176.3; HRMS calcd for [C₁₅H₂₁NO₄Na]⁺ [(M + Na)⁺]: 302.1363; found: 302.1359.

### (S)-2-((tert-Butoxycarbonyl)amino)-2-(4-methoxyphenyl)propanoic acid (26)

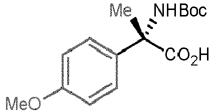

Following the **general procedure** [(*R*,*R*)-**FeBIP** (15 mol%), BocNHOMs (5 equiv.) and piperidine (7 equiv.) in o-DCB for 40 h], the α-amination of (±)-2-(4-methoxyphenyl)propanoic acid (36.0 mg, 0.2 mmol) gave **26** as a colorless foam in 79% yield (eluent: EtOAc/hexane = 1/4 with 0.2% HOAc, 46.5 mg) with 79% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 11.9 min, t₂ = 18.1 min]: [α]_{D}²⁵ = +49.4 (c 1.0, MeOH, 79% ee); ¹H NMR (300 MHz, CD₃OD) δ 1.22-1.53 (br, 9H), 1.89 (s, 3H), 3.78 (s, 3H), 6.78-7.00 (m, 2H), 7.31-7.53 (m, 2H); ¹³C NMR (75 MHz, CD₃OD) δ 24.0, 28.6, 55.7, 62.4, 80.6, 114.6, 128.3, 134.6, 156.6, 160.6, 176.4; HRMS calcd for [C₁₅H₂₁NO₅Na]⁺ [(M + Na)⁺]: 318.1312; found: 318.1310.

### (S)-2-((tert-Butoxycarbonyl)amino)-2-(4-chlorophenyl)propanoic acid (27)

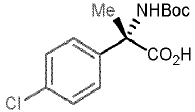

Following the **general procedure** [(*R*,*R*)-**FeBIP** (15 mol%), BocNHOMs (5 equiv.) and piperidine (7 equiv.) in o-DCB for 40 h], the α-amination of (±)-2-(4-chlorophenyl)propanoic acid (36.9 mg, 0.2 mmol) gave **27** as a colorless oil in 81% yield (eluent: EtOAc/hexane = 1/5 with 0.2% HOAc, 48.4 mg) with 89% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 6.5 min, t₂ = 7.8 min]: [α]_{D}²⁵ = +48.5 (c 1.0, MeOH, 89% ee); ¹H NMR (300 MHz, CD₃OD) δ 1.17-1.53 (br, 9H), 1.90 (s, 3H), 7.25-7.41 (m, 2H), 7.41-7.56 (m, 2H); ¹³C NMR (75 MHz, CD₃OD) δ 24.3, 28.6, 62.4, 80.8, 129.0, 129.2, 134.4, 141.7, 156.5, 175.7; HRMS calcd for [C₁₄H₁₈ClNO₄Na]⁺ [(M + Na)⁺]: 322.0817; found: 322.0813.

### (S)-2-((tert-Butoxycarbonyl)amino)-2-(4-(methoxycarbonyl)phenyl)propanoic acid (28)

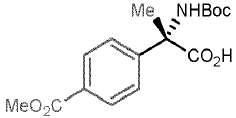

Following the **general procedure** [(*R*,*R*)-**FeBIP** (15 mol%), BocNHOMs (5 equiv.) and piperidine (7 equiv.) in o-DCB for 40 h], the α-amination of (±)-2-(4-(methoxycarbonyl)phenyl)propanoic acid (41.6 mg, 0.2 mmol) gave **28** as a colorless foam in 72% yield (eluent: EtOAc/hexane = 1/3 with 0.2% HOAc, 46.9 mg) with 90% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 14.8 min, t₂ = 19.9 min]: [α]_{D}²⁵ = +52.3 (c 1.0, MeOH, 90% ee); ¹H NMR (300 MHz, CD₃OD) δ 1.06-1.63 (br, 9H), 1.93 (s, 3H), 3.89 (s, 3H), 7.56-7.70 (m, 2H), 7.91-8.07 (m, 2H); ¹³C NMR (75 MHz, CD₃OD) δ 24.4, 28.6, 52.6, 62.8, 80.8, 127.6, 130.35, 130.38, 148.3, 156.4, 168.2, 175.4; HRMS calcd for [C₁₆H₂₁NO₆Na]⁺ [(M + Na)⁺]: 346.1261; found: 346.1254.

### (S)-2-((tert-Butoxycarbonyl)amino)-2-(naphthalen-2-yl)propanoic acid (29)

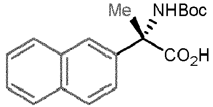

Following the **general procedure** [(*R*,*R*)-**FeBIP** (15 mol%), BocNHOMs (5 equiv.) and piperidine (7 equiv.) in o-DCB for 40 h], the α-amination of (±)-2-(naphthalen-2-yl)propanoic acid (40.0 mg, 0.2 mmol) gave **29** as a colorless foam in 68% yield (eluent: EtOAc/hexane = 1/5 with 0.2% HOAc, 42.6 mg) with 91% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 10.6 min, t₂ = 13.4 min]: [α]_{D}²⁵ = +53.1 (c 1.0, MeOH, 91% ee); ¹H NMR (300 MHz, CD₃OD) δ 1.11-1.57 (br, 9H), 2.04 (s, 3H), 7.40-7.53 (m, 2H), 7.61 (dd, *J*= 8.7, 2.0 Hz, 1H), 7.75-7.91 (m, 3H), 7.96 (d, *J=* 2.0 Hz, 1H); ¹³C NMR (75 MHz, CD₃OD) δ 24.2, 28.6, 63.0, 80.8, 125.2, 126.2, 127.2 (2C), 128.4, 128.9, 129.2, 134.1, 134.5, 140.0, 156.6, 176.1; HRMS calcd for [C₁₅H₂₁NO₄Na]⁺ [(M + Na)⁺]: 338.1363; found: 338.1359.

### (S)-2-((tert-Butoxycarbonyl)amino)-2-phenylbutanoic acid (30)

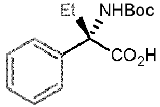

Following the **general procedure** [(*R*,*R*)-**FeBIP** (15 mol%), BocNHOMs (5 equiv.) and piperidine (7 equiv.) in o-DCB for 40 h], the α-amination of (±)-2-phenylbutanoic acid (32.8 mg, 0.2 mmol) gave **30** as a colorless foam in 85% yield (eluent: EtOAc/hexane = 1/6 with 0.2% HOAc, 47.6 mg) with 93% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 7.5 min, t₂ = 24.7 min]: [α]_{D}²⁵ = +66.8 (c 1.0, MeOH, 93% ee); ¹H NMR (300 MHz, CD₃OD) δ 0.90 (t, *J=* 7.2 Hz, 3H), 1.02-1.73 (br, 9H), 2.29-2.80 (m, 2H), 7.22-7.29 (m, 1H), 7.29-7.39 (m, 2H), 7.43-7.54 (m, 2H); ¹³C NMR (75 MHz, CD₃OD) δ 8.8, 27.9, 28.6, 66.7, 80.5, 127.2, 128.4, 129.2, 142.3, 156.0, 175.4; HRMS calcd for [C₁₅H₂₁NO₄Na]⁺ [(M + Na)⁺]: 302.1363; found: 302.1359.

### (S)-2-((tert-Butoxycarbonyl)amino)-2-phenylpentanoic acid (31)

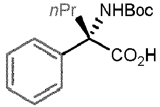

Following the **general procedure** [(*R*,*R*)-**FeBIP** (15 mol%), BocNHOMs (5 equiv.) and piperidine (7 equiv.) in o-DCB for 40 h], the α-amination of (±)-2-phenylpentanoic acid (35.6 mg, 0.2 mmol) gave **31** as a colorless foam in 76% yield (eluent: EtOAc/hexane = 1/6 with 0.2% HOAc, 44.5 mg) with 93% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 8.4 min, t₂ = 29.4 min]: [α]_{D}²⁵ = +60.7 (c 1.0, MeOH, 93% ee); ¹H NMR(300 MHz, CD₃OD) δ 0.99 (t, *J* = 7.3 Hz, 3H), 1.08-1.53 (m, 11H), 2.30-2.65 (m, 2H), 7.20-7.29 (m, 1H), 7.29-7.38 (m, 2H), 7.43-7.55 (m, 2H); ¹³C NMR (75 MHz, CD₃OD) δ 14.5, 18.7, 28.6, 37.3, 66.1, 80.5, 127.2, 128.4, 129.2, 142.5, 155.9, 175.5; HRMS calcd for [C₁₆H₂₄NO₄]⁺ [(M + H)⁺]: 294.1700; found: 294.1701.

### (S)-2-((tert-Butoxycarbonyl)amino)-3-methyl-2-phenylbutanoic acid (32)

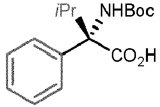

Following the **general procedure** [(*R*,*R*)-**FeBIP** (15 mol%), BocNHOMs (5 equiv.) and piperidine (7 equiv.) in o-DCB for 40 h], the α-amination of (±)-3-methyl-2-phenylbutanoic acid (35.6 mg, 0.2 mmol) gave **32** as a colorless foam in 64% yield (eluent: EtOAc/hexane = 1/6 with 0.2% HOAc, 37.8 mg) with 93% ee [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 5.4 min, t₂ = 18.1 min]: [α]_{D}²⁵ = +21.2 (c 1.0, MeOH, 93% ee); ¹H NMR (300 MHz, CD₃OD) δ 0.88 (d, *J=* 6.8 Hz, 3H), 0.91 (d, *J=* 6.8 Hz, 3H), 1.06-1.69 (br, 9H), 2.38-2.90 (br, 1H), 7.15-7.40 (m, 3H), 7.43-7.75 (m, 2H); ¹³C NMR (75 MHz, CD₃OD) δ 18.35, 18.41, 28.7, 36.8, 69.9, 80.5, 127.9, 128.5, 129.6, 140.3, 157.1, 175.2; HRMS calcd for [C₁₆H₂₃NO₄Na]⁺ [(M + Na)⁺]: 316.1519; found: 316.1512.

### (S)-2-((tert-Butoxycarbonyl)amino)-2-cyclohexyl-2-phenylacetic acid (33)

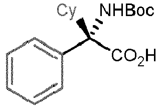

Following the **general procedure** [(*R*,*R*)-**FeBIP** (15 mol%), BocNHOMs (5 equiv.) and piperidine (7 equiv.) in o-DCB for 40 h], the α-amination of (±)-2-cyclohexyl-2-phenylacetic acid (43.7 mg, 0.2 mmol) gave **33** as a colorless foam in 59% yield (eluent: EtOAc/hexane = 1/6 with 0.2% HOAc, 39.2 mg) with 94% ee [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 6.0 min, t₂ = 25.6 min]: [α]_{D}²⁵ = +27.8 (c 1.0, MeOH, 94% ee); ¹H NMR (300 MHz, CD₃OD) δ 0.91-1.51 (m, 14H), 1.55-1.67 (m, 2H), 1.67-1.88 (m, 3H), 2.06-2.38 (br, 1H), 7.18-7.36 (m, 3H), 7.45-7.62 (m, 2H); ¹³C NMR (75 MHz, CD₃OD) δ 27.4, 27.7, 27.8, 28.7, 29.3, 29.4, 47.3, 69.9, 80.4, 127.8, 128.47, 128.52, 140.1, 157.0, 175.1; HRMS calcd for [C₁₉H₂₇NO₄Na]⁺ [(M + Na)⁺]: 356.1832; found: 356.1826.

### (S)-2-((tert-Butoxycarbonyl)amino)-2-(thiophen-3-yl)propanoic acid (34)

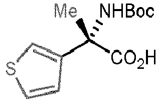

Following the **general procedure** [(*R*,*R*)-**FeBIP** (15 mol%), BocNHOMs (5 equiv.) and piperidine (7 equiv.) in o-DCB for 40 h], the α-amination of (±)-2-(thiophen-3-yl)propanoic acid (31.2 mg, 0.2 mmol) gave **34** as a colorless oil in 45% yield (eluent: EtOAc/hexane = 1/5 with 0.2% HOAc, 24.2 mg) with 88% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 9.2 min, t₂ = 15.2 min]: [α]_{D}²⁵ = +38.7 (c 1.0, MeOH, 88% ee); ¹H NMR (300 MHz, CD₃OD) δ 1.29-1.53 (br, 9H), 1.87 (s, 3H), 7.17 (dd, *J* = 5.1, 1.4 Hz, 1H), 7.35 (dd, *J*= 5.1, 3.1 Hz, 1H), 7.39 (dd, *J*= 3.1, 1.4 Hz, 1H); ¹³C NMR (75 MHz, CD₃OD) δ 24.9, 28.7, 60.9, 80.7, 122.9, 126.4, 127.5, 144.0, 156.7, 176.0; HRMS calcd for [C₁₂H₁₇NO₄SNa]⁺ [(M + Na)⁺]: 294.0770; found: 294.0768.

### (S)-2-((tert-Butoxycarbonyl)amino)-2-cyclohexylpropanoic acid (35)

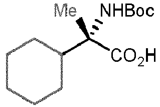

Following the **general procedure** [(*R*,*R*)-**FeBIP** (15 mol%), BocNHOMs (5 equiv.) and piperidine (7 equiv.) in CH₂Cl₂ for 40 h], the α-amination of (±)-2-cyclohexylpropanoic acid (31.2 mg, 0.2 mmol) gave **35** as a colorless foam in 43% yield (eluent: EtOAc/hexane = 1/7 with 0.2% HOAc, 23.1 mg) with 43% ee [DAICEL CHIRALPAK IG column, Agilent HPLC 1260, *i*PrOH/hexane = 5/95 (v/v) with 0.1% TFA, 1.0 mL/min, 25 °C, 210 nm; t₁ = 12.2 min, t₂ = 13.3 min]: [α]_{D}²⁵ = +23.1 (c 1.0, MeOH, 43% ee); ¹H NMR (300 MHz, CD₃OD) δ 0.99-1.53 (m, 17H), 1.63-1.92 (m, 6H); ¹³C NMR (75 MHz, CD₃OD) δ 19.4, 27.4, 27.7 (2C), 28.4, 28.75, 28.85, 46.2, 63.6, 80.4, 157.3, 177.5; HRMS calcd for [C₁₄H₂₅NO₄Na]⁺ [(M + Na)⁺]: 294.1676; found: 294.1673.

### (S)-2-((tert-Butoxycarbonyl)amino)-2-(11-oxo-6,11-dihydrodibenzo[b,e]oxepin-2-yl)acetic acid (36)

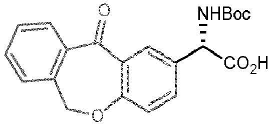

Following the **general procedure** [(*R*,*R*)-**FeBIP** (15 mol%), BocNHOMs (2 equiv.) and piperidine (4 equiv.) in o-DCB for 40 h], the α-amination of *isoxepac* (53.7 mg, 0.2 mmol) gave **36** as a colorless foam in 59% yield (eluent: EtOAc/hexane = 1/2 with 0.2% HOAc, 45.5 mg) with 90% ee [DAICEL CHIRALPAK IA column, Agilent HPLC 1260, *i*PrOH/hexane = 20/80 (v/v) with 0.1% TFA, 1.0 mL/min, 25 °C, 210 nm; t₁ = 13.0 min, t₂ = 16.0 min]: [α]_{D}²⁵ = +104.5 (c 1.0, MeOH, 90% ee); ¹H NMR (500 MHz, CD₃OD) δ 1.36-1.48 (br, 9H), 5.07-5.23 (br, 1H), 5.24 (s, 2H), 7.08 (d, *J* = 8.5 Hz, 1H), 7.47 (d, *J* = 7.5 Hz, 1H), 7.50 (td, *J* = 7.5, 1.1 Hz, 1H), 7.58 (dd, *J* = 8.5, 2.4 Hz, 1H), 7.62 (td, *J* = 7.5, 1.1 Hz, 1H), 7.84 (dd, *J* = 7.8, 1.0 Hz, 1H), 8.22 (d, *J* = 2.4 Hz, 1H); ¹³C NMR (125 MHz, CD₃OD) δ 28.7, 58.4, 74.6, 80.9, 122.3, 126.3, 129.2, 130.2, 130.3, 131.6, 132.8, 134.2, 135.8, 137.5, 141.6, 157.5, 162.7, 173.9, 192.1; HRMS calcd for [C₂₁H₂₁NO₆Na]⁺ [(M + Na)⁺]: 406.1261; found: 406.1252.

### (S)-2-((tert-Butoxycarbonyl)amino)-2-(2-((2,6-dichlorophenyl)amino)phenyl)acetic acid (37)

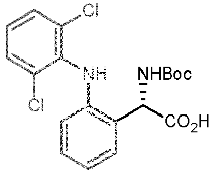

Following the **general procedure** [(*R*,*R*)-**FeBIP** (8 mol%), BocNHOMs (2 equiv.) and piperidine (4 equiv.) in o-DCB for 20 h], the α-amination of *diclofenac* (59.2 mg, 0.2 mmol) gave **37** as a light-yellow foam in 37% yield (eluent: EtOAc/hexane = 1/4 with 0.2% HOAc, 30.5 mg) with 83% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 10.7 min, t₂ = 21.5 min]: [α]_{D}²⁵ = +114.0 (c 1.0, MeOH, 83% ee); ¹H NMR (300 MHz, CD₃OD) δ 1.37-1.50 (br, 9H), 5.53-5.78 (br, 1H), 6.42 (dd, *J* = 8.1, 1.3 Hz, 1H), 6.95 (td, *J* = 7.6, 1.3 Hz, 1H), 7.06-7.15 (m, 2H), 7.28-7.37 (m, 1H), 7.42 (d, *J* = 8.1 Hz, 2H); ¹³C NMR (75 MHz, CD₃OD) δ 28.7, 55.0, 81.1, 118.5, 122.8, 126.1, 128.0, 128.6, 129.5, 130.1, 131.4, 138.7, 143.3, 157.6, 174.3; HRMS calcd for [C₁₉H₂₀Cl₂N₂O₄Na]⁺ [(M + Na)⁺]: 433.0692; found: 433.0686.

### (S)-2-((tert-Butoxycarbonyl)amino)-2-(1-(4-chlorobenzoyl)-5-methoxy-2-methyl-1H-indol-3-yl)acetic acid (38)

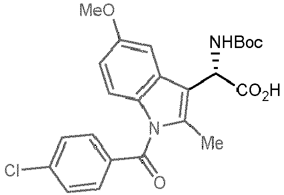

Following the **general procedure** [(*R*,*R*)-**FeBIP** (15 mol%), BocNHOMs (2 equiv.) and piperidine (4 equiv.) in o-DCB for 40 h], the α-amination of *indomethacin* (71.6 mg, 0.2 mmol) gave **38** as a light-yellow foam in 75% yield (eluent: EtOAc/hexane = 1/2 with 0.2% HOAc, 70.9 mg) with 70% ee [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 10.5 min, t₂ = 24.1 min]: [α]_{D}²⁵ = +86.9 (c 1.0, MeOH, 70% ee); ¹H NMR (300 MHz, CD₃OD) δ 1.38-1.50 (br, 9H), 2.39 (s, 3H), 3.78 (s, 3H), 5.30-5.59 (br, 1H), 6.64 (dd, *J* = 9.1, 2.5 Hz, 1H), 6.88 (d, *J* = 9.1 Hz, 1H), 7.14 (d, *J* = 2.5 Hz, 1H), 7.46-7.57 (m, 2H), 7.60-7.70 (m, 2H); ¹³C NMR (75 MHz, CD₃OD) δ 13.5, 28.7, 51.0, 56.1, 80.8, 103.0, 112.9, 115.8, 116.4, 129.9, 130.2, 132.3, 132.4, 135.3, 137.6, 140.4, 157.4, 157.6, 169.9, 173.9; HRMS calcd for [C₂₄H₂₅ClN₂O₆Na]⁺ [(M + Na)⁺]: 495.1293; found: 495.1289.

### (2S,4R)-2-((tert-Butoxycarbonyl)amino)-4-((3R,5R,8R,9S,10S,13R,14S,17R)-3-((tert-butyldimethylsilyl)oxy)-10,13-dimethylhexadecahydro-1H-cyclopenta[a]phenanthren-17-yl)pentanoic acid (39)

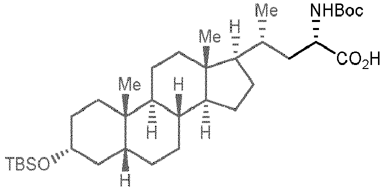

Following the **general procedure** [(*R*,*R*)-**FeBIP** (8 mol%), BocNHOMs (4 equiv.) and piperidine (6 equiv.) in CH₂Cl₂ for 20 h], the α-amination of O-TBS-protected *lithocholic acid* (98.2 mg, 0.2 mmol) gave **39** as a colorless foam in 43% yield (eluent: EtOAc/hexane = 1/7 with 0.2% HOAc, 52.4 mg) with 16/1 dr [DAICEL CHIRALPAK IG column, Agilent HPLC 1260, *i*PrOH/hexane = 5/95 (v/v) with 0.1% TFA, 1.0 mL/min, 25 °C, 210 nm; t₁ = 8.6 min, t₂ = 12.3 min]: [α]_{D}²⁵ = +51.9 (c 1.0, MeOH, 16/1 dr); ¹H NMR (300 MHz, CD₃OD) δ 0.07 (s, 6H), 0.69 (s, 3H), 0.90 (s, 9H), 0.94 (s, 3H), 1.04 (d, *J* = 6.4 Hz, 3H), 1.08-1.66 (m, 29H), 1.73-2.11 (m, 6H), 3.53-3.73 (m, 1H), 3.95-4.22 (m, 1H); ¹³C NMR (75 MHz, CD₃OD) δ -4.3, 12.5, 19.0, 19.7, 22.0, 24.0, 25.3, 26.5, 27.7, 28.4, 28.8, 29.4, 32.2, 35.0, 35.7, 36.6, 37.2, 38.1, 40.1, 41.6, 41.9, 43.6, 44.1, 53.6, 57.9, 58.0, 74.1, 80.4, 157.6, 176.6; HRMS calcd for [C₃₅H₆₃NO₅SiNa]⁺ [(M + Na)⁺]: 628.4368; found: 628.4363.

### (S)-2-((tert-Butoxycarbonyl)amino)-6-((4R,5S)-5-methyl-2-oxoimidazolidin-4-yl)hexanoic acid (40)

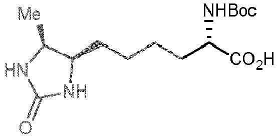

Following the **general procedure** [(*R*,*R*)-**FeBIP** (8 mol%), BocNHOMs (4 equiv.) and piperidine (6 equiv.) in CH₂Cl₂ for 20 h], the α-amination of *d-desthiobiotin* (42.9 mg, 0.2 mmol) gave **40** as a colorless foam in 40% yield (eluent: MeOH/CH₂Cl₂ = 1/10 with 0.2% HOAc, 26.5 mg) with 21/1 dr [DAICEL CHIRALPAK IG column, Agilent HPLC 1260, *i*PrOH/hexane = 20/80 (v/v) with 0.1% TFA, 1.0 mL/min, 25 °C, 210 nm; t₁ = 29.7 min, t₂ = 44.4 min]: [α]_{D}²⁵ = +14.4 (c 1.0, MeOH, 21/1 dr); ¹H NMR (300 MHz, CD₃OD) δ 1.11 (d, *J* = 6.4 Hz, 3H), 1.27-1.57 (m, 15H), 1.59-1.73 (m, 1H), 1.74-1.90 (m, 1H), 3.65-3.76 (m, 1H), 3.76-3.88 (m, 1H), 3.90-4.16 (br, 1H); ¹³C NMR (75 MHz, CD₃OD) δ 15.6, 26.8, 27.0, 28.7, 30.6, 32.8, 52.7, 54.9, 57.3, 80.4, 158.1, 166.2, 176.6; HRMS calcd for [C₁₅H₂₇N₃O₅Na]⁺ [(M + Na)⁺]: 352.1843; found: 352.1835.

### (S)-2-((tert-Butoxycarbonyl)amino)-2-(4-isobutylphenyl)propanoic acid (41)

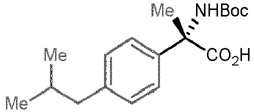

Following the **general procedure** [(*R*,*R*)-**FeBIP** (15 mol%), BocNHOMs (5 equiv.) and piperidine (7 equiv.) in o-DCB for 40 h], the α-amination of (±)-*ibuprofen* (41.3 mg, 0.2 mmol) gave **41** as a colorless oil in 87% yield (eluent: EtOAc/hexane = 1/6 with 0.2% HOAc, 56.0 mg) with 91% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 6.8 min, t₂ = 9.2 min]: [α]_{D}²⁵ = +47.0 (c 1.0, MeOH, 91% ee); ¹H NMR (300 MHz, CD₃OD) δ 0.90 (d, *J*= 6.7 Hz, 6H), 1.16-1.56 (br, 9H), 1.78-1.89 (m, 1H), 1.91 (s, 3H), 2.47 (d, *J* = 7.1 Hz, 2H), 7.07-7.19 (m, 2H), 7.33-7.46 (m, 2H); ¹³C NMR (75 MHz, CD₃OD) δ 22.7, 24.1, 28.6, 31.4, 45.9, 62.7, 80.7, 126.9, 130.0, 140.1, 142.2, 156.7, 176.3; HRMS calcd for [C₁₈H₂₇NO₄Na]⁺ [(M + Na)⁺]: 344.1832; found: 344.1826.

### (S)-2-(3-Benzoylphenyl)-2-((tert-butoxycarbonyl)amino)propanoic acid (42)

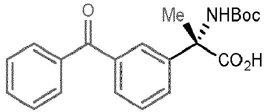

Following the **general procedure** [(*R*,*R*)-**FeBIP** (15 mol%), BocNHOMs (5 equiv.) and piperidine (7 equiv.) in o-DCB for 40 h], the α-amination of (±)-*ketoprofen* (50.9 mg, 0.2 mmol) gave **42** as a colorless oil in 88% yield (eluent: EtOAc/hexane = 1/3 with 0.2% HOAc, 65.4 mg) with 90% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 26.4 min, t₂ = 33.2 min]: [α]_{D}²⁵ = +33.6 (c 1.0, MeOH, 90% ee); ¹H NMR (300 MHz, CD₃OD) δ 1.17-1.54 (br, 9H), 1.93 (s, 3H), 7.45-7.57 (m, 3H), 7.58-7.72 (m, 2H), 7.72-7.84 (m, 3H), 7.90-7.99 (m, 1H); ¹³C NMR (75 MHz, CD₃OD) δ 24.6, 28.6, 62.7, 80.8, 129.0, 129.4, 129.5, 130.1, 131.0, 131.8, 133.8, 138.6, 138.7, 143.3, 156.5, 175.6, 198.2; HRMS calcd for [C₂₁H₂₃NO₅Na]⁺ [(M + Na)⁺]: 392.1468; found: 392.1460.

### (S)-2-((tert-Butoxycarbonyl)amino)-2-(6-methoxynaphthalen-2-yl)propanoic acid (43)

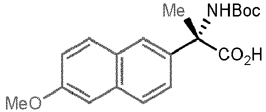

Following the **general procedure** [(*R*,*R*)-**FeBIP** (15 mol%), BocNHOMs (5 equiv.) and piperidine (7 equiv.) in o-DCB for 40 h], the α-amination of (±)-*naproxen* (46.1 mg, 0.2 mmol) gave **43** as a colorless foam in 65% yield (eluent: EtOAc/hexane = 1/3 with 0.2% HOAc, 45.1 mg) with 85% ee [DAICEL CHIRALPAK IG column, Agilent HPLC 1260, *i*PrOH/hexane = 10/90 (v/v) with 0.1% TFA, 1.0 mL/min, 25 °C, 210 nm; t₁ = 14.9 min, t₂ = 20.6 min]: [α]_{D}²⁵ = +68.6 (c 1.0, MeOH, 85% ee); ¹H NMR (300 MHz, CD₃OD) δ 1.00-1.69 (br, 9H), 2.01 (s, 3H), 3.88 (s, 3H), 7.01-7.15 (m, 1H), 7.15-7.29 (m, 1H), 7.44-7.64 (m, 1H), 7.64-7.81 (m, 2H), 7.81-8.00 (m, 1H); ¹³C NMR (75 MHz, CD₃OD) δ 24.1, 28.6, 55.7, 62.9, 80.7, 106.4, 120.0, 125.6, 126.0, 127.9, 129.9, 130.6, 135.4, 137.6, 156.7, 159.5, 176.3; HRMS calcd for [C₁₉H₂₃NO₅Na]⁺ [(M + Na)⁺]: 368.1468; found: 368.1463.

### 4. Determining the Absolute Configuration of the α-Amino Acids

The absolute configuration of α-amino acids **1** and **23** was assigned by HPLC analysis using enantiopure (*S*)-Boc-Phg-OH and (*S*)-Boc-Tle-OH received from commercial sources.

In order to determine the absolute configuration of α,α-disubstituted α-amino acid derived from (*R*,*R*)-**FeBIP** catalyzed α-amination, enantioenriched **24** (91% ee) was converted to *N*-Troc α-methyl-α-phenylglycine, whose absolute configuration has been assigned in the literature (19).

**Procedures for converting enantioenriched 24 into *N*-Troc α-methyl-α-phenylglycine:** to a solution of **24** (26 mg, 0.1 mmol, 91% ee) in dichloromethane (2 mL) at room temperature was added trifluoroacetic acid (0.1 mL) in one portion. The reaction mixture was stirred at room temperature for 1 hour. After completion, the solvent was evaporated under reduced pressure. The residue was redissolved in a mixed solvent of tetrahydrofuran/water (v/v = 1/1, 2 mL). Na₂CO₃ (21 mg, 0.2 mmol, 2 equiv.) was added, and the mixture was cooled to 0 °C before addition of 2,2,2-trichloroethyl chloroformate (20 µL, 0.15 mmol, 1.5 equiv.) dropwise. The reaction mixture was warmed to room temperature and was stirred for 1 hour. After completion, the reaction mixture was acidified with hydrochloric acid (aqueous solution, 1 M, 5 mL). The mixture was extracted with EtOAc for three times. The combined organic layer was dried over anhydrous sodium sulfate. After filtration, the solvent was evaporated under reduced pressure, and the residue was purified by column chromatography (silica gel, eluent: EtOAc/hexane = 1/5 with 0.2% HOAc) to afford *N*-Troc α-methyl-α-phenylglycine as a colorless oil (16 mg, 47% yield): ¹H NMR (300 MHz, CD₃OD) δ 1.98 (s, 3H), 4.72 (d, *J* = 12.2 Hz, 1H), 4.75 (d, *J* = 12.2 Hz, 1H), 7.14-7.42 (m, 3H), 7.44-7.62 (m, 2H); ¹³C NMR (75 MHz, CD₃OD) δ 23.5, 63.2, 75.2, 97.1, 127.2, 128.8, 129.3, 141.9, 154.8, 175.6. The NMR data matched those reported in the literature (23).

### References

1. M. A. T. Blaskovich, J. Med. Chem. 59, 10807-10836 (2016).
2. F. Agostini, J.-S. Voller, B. Koksch, C. G. Acevedo-Rocha, V. Kubyshkin, N. Budisa, Angew. Chem. Int. Ed. 56, 9680-9703 (2017).
3. D. D. Young, P. G. Schultz, ACS Chem. Biol. 13, 854-870 (2018).
4. H. Yokoo, M. Hirano, T. Misawa, Y. Demizu, ChemMedChem 16, 1226-1233 (2021).
5. R. O. Dafydd et al., Science 374, 1586-1593 (2021).
6. C. Nájera, J. M. Sansano, Chem. Rev. 107, 4584-4671 (2007).
7. Y.-P. Xue, C.-H. Cao, Y.-G. Zheng, Chem. Soc. Rev. 47, 1516-1561 (2018).
8. J. M. Janey, Angew. Chem. Int. Ed. 44, 4292-4300 (2005).
9. A. M. R. Smith, K. K. Hii, Chem. Rev. 111, 1637-1656 (2011).
10. A. de la Torre, V. Tona, N. Maulide, Angew. Chem. Int. Ed. 56, 12416-12423 (2017).
11. S.-i. Yamada, T. Oguri, T. Shioiri, J. Chem. Soc., Chem. Commun. 623 (1972).
12. L. C. Morrill, T. Lebl, A. M. Z. Slawin, A. D. Smith, Chem. Sci. 3, 2088-2093 (2012).
13. T. Morisawa, M. Sawamura, Y. Shimizu, Org. Lett. 21, 7466-7469 (2019).
14. Y. Park, Y. Kim, S. Chang, Chem. Rev. 117, 9247-9301 (2017).
15. F. Collet, C. Lescot, P. Dauban, Chem. Soc. Rev. 40, 1926-1936 (2011).
16. M. Ju, J. M. Schomaker, Nat. Rev. Chem. 5, 580-594 (2021).
17. X. Xiao et al., Angew. Chem. Int. Ed. 55, 11897-11901 (2016).
18. Y. Wang, H. Liu, B. Li, B. Wang, Adv. Synth. Catal. 361, 1564-1569 (2019).
19. C.-X. Ye, X. Shen, S. Chen, E. Meggers, Nat. Chem. 14, 566-573 (2022).
20. A. Isidro-Llobet, M. Alvarez, F. Albericio, Chem. Rev. 109, 2455-2504 (2009).
21. G. Cardillo, L. Gentilucci, I. Ratera Bastardas, A. Tolomelli, Tetrahedron 54, 8217-8222 (1998).
22. J. A. Stafford, S. S. Gonzales, D. G. Barrett, E. M. Suh, P. L. Feldman, J. Org. Chem. 63, 10040-10044 (1998).
23. H. Lebel, K. Huard, Org. Lett. 9, 639-642 (2007).
24. K. Huard, H. Lebel, Chem. Eur. J. 14, 6222-6230 (2008).
25. Masruri, A. C. Willis, M. D. McLeod, J. Org. Chem. 77, 8480-8491 (2012).
26. P. Patel, S. Chang, Org. Lett. 16, 3328-3331 (2014).
27. S. Hanessian, S. Johnstone, J. Org. Chem. 64, 5896-5903 (1999).
28. C. Toniolo, F. Formaggio, B. Kaptein, Q. B. Broxterman, Synlett 1295-1310 (2006).
29. M. Tanaka, Chem. Pharmaceut. Bull. 55, 349-358 (2007).
30. C. Cativiela, M. D. Diaz-de-Villegas, Tetrahedron: Asymmetry 9, 3517-3599 (1998).
31. H. Vogt, S. Bräse, Org. Biomol. Chem. 5, 406-430 (2007).
32. A. E. Metz, M. C. Kozlowski, J. Org. Chem. 80, 1-7 (2015).

## Claims

1. Method for preparing α-amino acids and α-deuterated α-amino acids, comprising the step of reacting a carboxylic acid with a carbamate of the formula PG-NH-X, wherein PG is a protecting group and X is a leaving group, in the presence of a catalyst, wherein the catalyst provides an enantioselective single-step α-amination of the carboxylic acid.

2. The method according to claim 1, wherein the leaving group is a sulfonate.

3. The method according to claim 2, wherein the sulfonate is selected from the group of toluenesulfonate, isopropylsulfonate and mesylate.

4. The method according to any of the preceding claims, wherein the protecting group is an electron withdrawing protecting group.

5. The method according to claim 4, wherein the protecting group is selected from the group consisting of the CO₂Me group, the CO₂CH₂CCl₃ (Troc) group and the butyloxy carbonyl (Boc) group, the benzylcarbonyl (Cbz) group, the allyloxycarbonyl (Alloc) group, among other amine protecting groups which form a carbamate.

6. The method according to any of the preceding claims, wherein the carboxylic acid has at least one hydrogen or deuterium at the α-C-atom and one or two groups bound to the α-C-atom.

7. The method according to any of the preceding claims, wherein the catalyst is a chiral non-racemic catalyst providing chiral non-racemic α-amino acids and chiral non-racemic α-deutereated amino acids or wherein the catalyst is a achiral or racemic catalyst providing raccemic α-amino acids and racemic α-deutereated amino acids.

8. The method according to claim 7, wherein the chiral non-racemic catalyst is a chiral non-racemic iron catalyst.

9. The method according to claim 8, wherein the chiral non-racemic iron catalyst is a chiral non-racemic iron(II) catalyst.

10. The method according to any of claims 7 to 9, wherein the catalyst contains two monodentate ligands.

11. The method according to claim 10, wherein the monodentate ligand is selected from the group consisting of acetonitrile, triflate or other sulfates, acetate or other carboxylates, chloride, bromide, iodide, pseudohalides and other labile monodentate ligands.

12. The method according to any of claims 7 to 11, wherein the catalyst contains one tetradentate ligand.

13. The method according claim 12, wherein the tetradentate ligand contains four N-atoms to bind to a central atom.

14. The method according to any of claims 7 to 13, wherein the catalyst is selected from the group consisting of *(R*,*R)*-Fe1, (R,R)-Fe2 and (*R*,*R*)-FeBIP or their (*R*,*R*)-enantiomers.

15. Use of a catalyst in the method as defined in any of claims 1 to 14.
